# EUROPÄISCHE PATENTSCHRIFT

(11) **EP 1 802 559 B1**
(45) Veröffentlichungstag und Bekanntmachung des Hinweises auf die Patenterteilung: **01.08.2012**
(21) Anmeldenummer: 05796208.6
(22) Anmeldetag: 13.10.2005
(51) Int. Cl.: C07C 5/05, C07C 11/08, C07C 6/04, C07C 11/10, C07C 11/107

(54) **VERFAHREN ZUR HERSTELLUNG EINES C4-OLEFIN-GEMISCHES DURCH SELEKTIVHYDRIERUNG UND METATHESEVERFAHREN ZUR VERWENDUNG DIESES STROMS**
METHOD FOR PRODUCING A C4-OLEFIN MIXTURE BY MEANS OF SELECTIVE HYDROGENATION, AND METATHESIS METHOD FOR USING SAID FLOW
PROCEDE POUR PRODUIRE UN MELANGE D'OLEFINES C4 PAR HYDROGENATION SELECTIVE ET PROCEDE DE METATHESE POUR UTILISER CE FLUX

(30) Priorität: 13.10.2004 DE 102004049940
(43) Veröffentlichungstag der Anmeldung: 04.07.2007
(73) Patentinhaber: BASF SE, 67056 Ludwigshafen (DE)
(72) Erfinder: STEPHAN, Jürgen, 68163 Mannheim (DE); BRODHAGEN, Andreas, B-3060 Bierbeeg (BE); SCHUBERT, Markus, 67063 Ludwigshafen (DE); POPLOW, Frank, 67065 Ludwigshafen (DE); RÖPER, Michael, 67157 Wachenheim (DE); HILL, Thomas, 67071 Ludwigshafen (DE)
(86) Internationale Anmeldenummer: PCT/EP2005/011027
(87) Internationale Veröffentlichungsnummer: WO 2006/040160

(56) Entgegenhaltungen:
- WO-A-03/070669
- DE-A1- 10 039 995

## Beschreibung

Die vorliegende Erfindung betrifft C4-Olefin-Gemische und ein Verfahren zu ihrer Herstellung. Darüber hinaus betrifft die vorliegende Erfindung ein Verfahren zur Herstellung eines 2-Penten und/der 3-Hexen enthaltenden Olefin-Gemisches durch Metathese des C4-Olefin-Gemisches an einem Metathese-Katalysator.

Die Olefin-Metathese hat sich in den letzten Jahren zu einem äußerst wertvollen Tool in der organischen Synthese entwickelt. Auch im technischen Maßstab haben sich eine Reihe von Anwendungen etabliert, beispielsweise der Phillips-Prozess zur Herstellung von Propen durch Ethenolyse (metathetische Spaltung mittels Ethen) von 2-Buten oder das Verfahren der Shell AG (SHOP) zur Herstellung von internen Olefinen, wobei der Metathese-Schritt einen wichtigen Baustein darstellt.

Ein jüngeres Beispiel ist die Herstellung von α,ω-Dienen durch Ethenolyse von Cycloolefinen (FEAST-Prozess der Shell AG).

Der wertvollen Anwendungsbreite der Metathese-Reaktion steht allerdings ein wesentlicher Punkt entgegen, der die Entwicklung der technischen Prozesse seit langem stark beeinflusst: Metathese-Katalysatoren desaktivieren im Vergleich zu anderen technisch angewendeten Katalysatorsystemen relativ rasch. Aufgrund der verwendeten, oft teuren Übergangsmetall-Katalysatoren, welche Methatese-aktiv sind, ist es wünschenswert, die Desaktivierung, die beispielsweise auf Feed-Verunreinigungen zurück zu führen ist, zu reduzieren oder zu vermeiden.

Die Ursache für die Desaktivierung von Metathese-Katalysatoren ist in der Literatur bereits intensiv diskutiert worden. Beispiele hierfür sind J. Mol. Cat. 1991, 65, Seiten 39 - 50 (Commereuc et al.), Catalysis today 1999, 51, Seiten 289 - 299 (J. C. Mol) und J. Mol. Cat. 1991, 65, Seiten 219 - 235 (J. C. Mol).

Prinzipiell werden in der Literatur zwei Wege der Desaktivierung postuliert, nämlich ein intrinsischer Weg, der immer präsent ist, sowie ein Desaktivierungs-Mechanismus, der durch bestimmte Verunreinigungen im Feed-Strom verursacht wird. Diese Verunreinigungen im Feed-Strom können einen reversiblen Effekt haben oder als permanente Gifte wirken.

Insbesondere werden als desaktivierende Substanzen in der Literatur Isobuten und 1,3-Butadien genannt, da sie durch kationische Mechanismen zur Bildung von Oligomeren neigen, die als Diffusionsbarriere fungieren. Weiterhin werden polare, basische Komponenten als eine wichtige Klasse von desaktivierenden Substanzen genannt. Dieser Einfluss ist bekannt und wird im Stand der Technik durch die Verwendung adsorptiver Feed-Reinigungen - Schutzbetten (z. B. Molsiebe) - vermieden. Eine detaillierte Untersuchung über den Einfluss sauerstoffhaltiger Verbindungen auf Metathese-Katalysatoren findet sich bei J. A. K. du Plissis, J. Mol. Cat. A: Chemical, 1989, 133, Seiten 181 - 186. Als adsorptive Feed-Reinigung kommen insbesondere Zeolithe oder Aluminiumoxide in Frage.

Der Einfluss von acetylenischen Verbindungen und 1,3-Dienen wird ebenfalls als wesentlich beschrieben, siehe EP 742 234 A1. Durch diese Komponenten erfolgt eine erhebliche Desaktivierung, der entgegengewirkt werden muss, um einen wirtschaftlichen Betrieb der Reaktion zu gewährleisten.

Als Gegenmaßnahme gegen die im C4-Feed präsenten 1,3-Diene und acetylenischen Verbindungen wird eine Selektivhydrierung beschrieben, die darüber hinaus allerdings 1-Buten zu 2-Buten isomerisiert. Diese Maßnahme bietet daher jedoch keine Abhilfe zur selektiven Entfernung von 1,2-Dienen unter gleichzeitigem Erhalt eines hohen 1-Buten-Gehalts. Dieses ist aber notwendig, wenn das Ziel der Metathese-Reaktion ist, große Mengen an 3-Hexen zu produzieren. In der WO 03/070669 A2 wird auf die Notwendigkeit einer selektiven Hydrierung des C4-Schnittes hingewiesen. Dabei werden sogenannte "low isom"-Bedingungen genannt, die bei möglichst geringer Isomerisierung von C-C-Doppelbindungen zu Restgehalten von 0 bis 50 ppm 1,3-Butadien führen.

DE 100 13 253 A1 beschreibt geeignete Vorbehandlungen für C4-Ströme, die für die Metathese eingesetzt werden. Dabei wird die Abtrennung von 1,3-Butadien und acetylenischen Verbindungen durch Extraktion und/oder Selektivhydrierung erreicht. Der Grenzwert für die Summe an Dienen wird in der DE 100 13 253 A1 als kleiner 10 ppm definiert.

Durch die in den zuvor erwähnten Schriften WO 03/070669 A2 und DE 100 13 253 A1 beschriebenen Vorbehandlungen werden jedoch keine Ströme erhalten, welche gleichzeitig einen geringen Anteil an desaktivierenden Komponenten und einen hohen Gehalt an 1-Buten aufweisen.

Aufgabe der vorliegenden Erfindung ist es somit, ein C4-Olefin-Gemisch bereitzustellen, welches einen hohen Gehalt an 1-Buten aufweist und ohne starke Desaktivierung von Metathese-Katalysatoren in Metathese-Reaktionen eingesetzt werden kann.

Erfindungsgemäß wird diese Aufgabe durch ein C4-Olefin-Gemisch gelöst, bei dem das Gemisch Restgehalte von 100 bis 500 ppm 1,3-Butadien aufweist, wobei jedoch die besonders stark desaktivierenden 1,2-Diene bis auf 1 bis 10 ppm abgesenkt werden. Der Gehalt an 1-Buten in dem C4-Olefin-Gemisch, bezogen auf das Gemisch, beträgt dabei mindestens 30 Gew.-%, besonders bevorzugt mindestens 40 Gew.-%, insbesondere mindestens 50 Gew.-%. Erfindungsgemäß wurde gefunden, dass der desaktivierende Einfluss von 1,2-Dienen (z. B. Propadien oder 1,2-Butadien) auf Metathese-Katalysatoren den Einfluss von konjugierten Dienen (z. B. 1,3-Butadien) oder Alkinen (z. B. 1-Butin) und Alkinenen (z. B. Butenin) deutlich übersteigt.

Die vorliegende Erfindung betrifft somit ein C4-Olefin-Gemisch.

Das erfindungsgemäße C4-Olefin-Gemisch ist dann durch einen Gehalt an 1,3-Butadien von 100 bis 500 ppm, vorzugsweise 110 bis 400 ppm, besonders bevorzugt 120 bis 300 ppm, und einen Gehalt an kumulierten Dienen wie Propadien, 1,2-Butadien, 1,2-Pentadien oder 2,3-Pentadien von 1 bis 10 ppm, besonders bevorzugt von 2 bis 10 ppm, gekennzeichnet.

In einer besonderen Ausführungsform handelt es sich bei den kumulierten Dienen um kumulierte 1,2-Diene.

Das Verhältnis von 1-Buten zu 2-Buten im C4-Olefin-Gemisch beträgt vorzugsweise 1,2 bis 2,0, besonders bevorzugt 1,3 bis 1,6.

Das erfindungsgemäße C4-Olefin-Gemisch kann 1-Buten, trans-2-Buten, cis-2-Buten, Isobuten, Isobutan und n-Butan enthalten.

Das erfindungsgemäße C4-Olefin-Gemisch kann mittels selektiver Bedingungen einer Hydrierung, die es erlauben, kumulierte Diene unter 10 ppm abzusenken und gleichzeitig nur wenig 1-Buten zu isomerisieren, ausgehend von Austrägen aus Steamcrackern erhalten werden. Hierbei wird ein für die Metathese von C4-Olefin-Strömen zu 2-Penten und/oder 3-Hexen enthaltenden Gemischen besonders vorteilhafter Ausgangsstrom hergestellt.

Die erfindungsgemäßen C4-Olefin-Gemische können also durch selektive Hydrierung von Austrägen aus Steamcrackern erhalten werden.

Daher betrifft die vorliegende Erfindung auch ein Verfahren zur Herstellung des vorstehenden C4-Olefin-Gemisches durch Hydrierung eines aus Steamcrackern stammenden C4-Stromes, wobei in der ersten Hydrierstufe die Hydrierung an einem Katalysator durchgeführt wird, der mindestens ein Metall der VIII. Gruppe des Periodensystems der Elemente als Hydriermetall und zusätzlich einen Promotor auf einem oxidischen Träger umfasst, wobei mindestens 80 % des Metalls der VIII. Gruppe des Periodensystems der Elemente in einer Schicht zwischen der Oberfläche des Katalysators und einer Eindringtiefe, die maximal 80 % des Radius des Katalysators, gerechnet von der Oberfläche des Katalysators, entspricht, im Wesentlichen homogen und der Promotor über den gesamten Querschnitt des Katalysators im Wesentlichen homogen verteilt vorliegt. Die. Hydrierung wird dabei vorzugsweise in mindestens zwei Hydrierstufen durchgeführt.

Das erfindungsgemäße Verfahren ist in einer bevorzugten Ausführungsform dadurch gekennzeichnet, dass ein aus Steamcrackern stammender C4-Strom in mindestens zwei Stufen hydriert wird.

In einer bevorzugten Ausführungsform weist der Katalysator einen Durchmesser von 2,5 bis 10 mm auf, wobei mindestens 80% des Metalls der VIII. Gruppe des Periodensystems der Elemente in einer Schicht zwischen der Oberfläche des Katalysators und einer Eindringtiefe von maximal 1000 µm, gerechnet von der Oberfläche des Katalysators, im Wesentlichen homogen und der Promotor über den gesamten Querschnitt im Wesentlichen homogen verteilt vorliegt.

Erfindungsgemäß ist somit ein Katalysator vorgesehen, bei dem das Metall der VIII. Gruppe des Periodensystems der Elemente eine Schalenstruktur in dem Katalysator ausbildet, während der Promotor durchgetränkt ist.

Die Bezeichnung der Gruppen des Periodensystems der Elemente erfolgt gemäß der CAS-Nomenklatur (*chemical abstracts service*).

Der erfindungsgemäße Katalysator weist einen Durchmesser von 2,5 bis 10 mm auf. In bevorzugten Ausführungsformen des erfindungsgemäßen Katalysators beträgt der Durchmesser 2,5 bis 5 mm, insbesondere 2,5 bis 3,5 mm.

In dem erfindungsgemäßen Katalysator liegen mindestens 80%, vorzugsweise mindestens 90%, besonders bevorzugt mindestens 95%, insbesondere mindestens 98%, speziell 100%, des Metalls der VIII. Gruppe des Periodensystems der Elemente in einer Schicht zwischen der Oberfläche des Katalysators und einer Eindringtiefe von maximal 1000 µm, gerechnet von der Oberfläche des Katalysators, im Wesentlichen homogen verteilt vor.

Der erfindungsgemäße Katalysator enthält ein Metall der VIII. Gruppe des Periodensystems der Elemente (Fe, Ru, Os, Co, Rh, Ir, Ni, Pd, Pt). In einer bevorzugten Ausführungsform der vorliegenden Erfindung handelt es sich um Palladium.

Der erfindungsgemäße Katalysator enthält darüber hinaus mindestens einen Promotor. Beispielsweise kann es sich hierbei um weitere Metalle der VIII., IB. und IIB. Gruppe des Periodensystems der Elemente handeln (Cu, Ag, Au, Zn, Cd, Hg). In einer bevorzugten Ausführungsform enthalten die enthalten die erfindungsgemäßen Katalysatoren neben dem Metall der VIII. Gruppe des Periodensystems der Elemente noch mindestens ein Metall aus der IB. Gruppe des Periodensystems der Elemente. Besonders bevorzugt ist hierbei Silber.

In einer besonders bevorzugten Ausführungsform enthält der erfindungsgemäße Katalysator Palladium und Silber.

Der erfindungsgemäße Katalysator kann beliebige Formen, beispielsweise Stränge, Hohlstränge, Tabletten, Ringe, sphärische Teilchen oder Kugeln, aufweisen. Bevorzugt ist es, wenn der erfindungsgemäße Katalysator als Strang ausgebildet ist.

Die Metalle können in reiner metallischer Form vorliegen, aber auch in Form von Verbindungen, beispielsweise in Form von Metalloxiden. Unter den Betriebsbedingungen eines Hydrierverfahrens liegen sie im Allgemeinen in Form von Metallen vor. Die Umwandlung etwaiger Oxide in Metalle kann auf dem Fachmann bekannte Weise vor dem Einsatz des Katalysators in einem Hydrierverfahren in oder außerhalb eines Hydrierreaktors, beispielsweise durch Vorreduzierung und, falls für Manipulationen mit dem vorreduzierten Katalysator erforderlich oder vorteilhaft, anschließende oberflächliche Passivierung erfolgen.

Der Gehalt des Katalysators an Metall oder Metallen der VIII. Gruppe des Periodensystems, insbesondere Palladium, beträgt vorzugsweise mindestens 0,01 Gew.-%, besonders bevorzugt mindestens 0,1 Gew.-%, insbesondere mindestens 0,15 Gew.-%. Vorzugsweise liegt dieser Gehalt bei höchstens 5 Gew.-%, besonders bevorzugt höchstens 1 Gew.-%, insbesondere höchstens 0,6 Gew.-%. Niedrigere und höhere Gehalte sind zwar möglich, aber im Normalfall wegen zu niedriger Aktivität oder zu hohen Rohstoffkosten wirtschaftlich unbefriedigend. In einer besonders bevorzugten Ausführungsform wird nur ein Hydriermetall, insbesondere Palladium, verwendet.

Das Verhältnis der Mengen von Hydriermetall der VIII. Gruppe des Periodensystems der Elemente und Zusatz- oder Dotierstoffen ist ein im Einzelfall zu optimierender Parameter. Vorzugsweise beträgt das Atomverhältnis von Metall der VIII. Gruppe des Periodensystems der Elemente, besonders bevorzugt Palladium, zu dem Promotor, besonders bevorzugt Silber, vorzugsweise 0,1 - 10, besonders bevorzugt 2 - 7, insbesondere 2,5 - 6.

Der oxidische Träger des erfindungsgemäßen Hydrierkatalysators ist bevorzugt Aluminiumoxid, besonders bevorzugt in einer Mischung aus δ-, θ- und α-Aluminiumoxid. Der Träger kann neben unvermeidbaren Verunreinigungen in gewissem Umfang auch andere Zusätze enthalten. Beispielsweise können andere anorganische Oxide wie Oxide von Metallen der IIA., IIIB., IVB., IIIA. und IVA. Gruppe des Periodensystems der Elemente enthalten sein, insbesondere Siliziumdioxid, Titandioxid, Zirkondioxid, Zinkoxid, Magnesiumoxid, Natriumoxid und Kalziumoxid. Der maximale Gehalt des Trägers an solchen von Aluminiumoxid verschiedenen Oxiden ist vom tatsächlich vorhandenen Oxid abhängig, aber im Einzelfall anhand des Röntgenbeugungsdiagramms des Hydrierkatalysators zu ermitteln, da eine Änderung der Struktur mit einer signifikanten Änderung des Röntgenbeugungsdiagramms einher geht. Im Allgemeinen liegt der Gehalt an solchen, von Aluminiumoxid verschiedenen Oxiden, unterhalb von 50 Gew.-%, vorzugsweise unterhalb von 30 Gew.-%, besonders bevorzugt unterhalb von 10 Gew.-%. Der Reinheitsgrad des Aluminiumoxids liegt vorzugsweise höher als 99%.

Zur Herstellung des Trägers wird ein geeigneter aluminiumhaltiger Rohstoff, vorzugsweise Böhmit, mit einem Peptisierungsmittel, wie Wasser, verdünnter Säure oder verdünnter Base, peptisiert. Als Säure wird beispielsweise eine Mineralsäure, wie etwa Salpetersäure, oder eine organische Säure, wie etwa Ameisensäure, verwendet. Als Base wird vorzugsweise eine anorganische Base, wie etwa Ammoniak, verwendet. Die Säure oder Base wird im Allgemeinen in Wasser gelöst. Vorzugsweise werden als Peptisierungsmittel Wasser oder verdünnte wässerige Salpetersäure verwendet. Die Konzentration des nicht-wässerigen Anteils im Peptisierungsmittel beträgt im Allgemeinen 0 - 10 Gew.-%, vorzugsweise 0 - 7 Gew.-%, besonders bevorzugt 0 - 5 Gew.-%. Im Anschluss an die Peptisierung wird der Träger verformt getrocknet und kalziniert.

Böhmit (γ-AlO(OH)) ist ein verbreitetes Handelsprodukt, kann aber auch in bekannter Weise unmittelbar vor der eigentlichen Trägerherstellung durch Fällung aus einer Lösung eines Aluminiumsalzes, beispielsweise Aluminiumnitrat, mit einer Base, Abtrennen, Waschen, Trocknen und Kalzinieren des gefällten Feststoffes hergestellt werden. Vorteilhafterweise wird Böhmit in der Form eines Pulvers verwendet. Ein geeignetes handelsübliches Böhmit-Pulver ist beispielsweise Versal^{®} 250, das von UOP erhältlich ist. Der Böhmit wird mit dem Peptisierungsmittel behandelt, indem er mit dem Peptisierungsmittel angefeuchtet und intensiv durchmischt wird, beispielsweise in einem Kneter, Mischer oder Kollergang. Die Peptisierung wird fortgesetzt, bis die Masse gut verformbar ist. Anschließend wird die Masse mittels üblicher Methoden zu den gewünschten Trägerformkörpern verformt, beispielsweise durch Strangpressen, Extrudieren, Tablettieren oder Agglomerieren. Zur Verformung ist jede bekannte Methode geeignet. Falls erforderlich oder vorteilhaft können übliche Zusätze verwendet werden. Beispiele für solche Zusätze sind Extrudier- oder Tablettierhilfsmittel, wie Polyglykole oder Graphit.

Es ist ferner möglich, der Trägerrohmasse vor der Verformung Zusätze beizumischen, die in bekannter Weise als Ausbrennstoffe die Porenstruktur des Trägers nach der Kalzination beeinflussen, beispielsweise Polymere, Faserstoffe, natürliche Ausbrennstoffe, wie Nussschalenmehle, oder andere übliche Zusätze. Bevorzugt ist die Verwendung von Böhmit in einer Korngrößenverteilung und die Zugabe von Ausbrennstoffen, die zu einer Porenradienverteilung des fertigen Trägers führt, bei der 50 - 90 Vol.-% des gesamten Porenvolumens in Form von Poren mit einem mittleren Durchmesser im Bereich von 0,01 - 0,1 µm und 10 - 50 Vol.% des gesamten Porenvolumens in Form von Poren mit einem mittleren Durchmesser im Bereich von 0,1 - 1 µm vorliegen. Die hierzu notwendigen Maßnahmen sind dem Fachmann an sich bekannt.

Im Anschluss an die Verformung werden die Formkörper in üblicher Weise getrocknet, im Allgemeinen bei einer Temperatur oberhalb von 60°C, vorzugsweise oberhalb von 80 °C, besonders bevorzugt oberhalb von 100°C, insbesondere bei einer Temperatur im Bereich von 120 - 300 °C. Die Trocknung wird fortgesetzt, bis in Formkörpern vorhandenes Wasser im Wesentlichen vollständig aus den Formkörpern entwichen ist, was im Allgemeinen nach einigen Stunden der Fall ist. Übliche Trocknungsdauem liegen im Bereich von 1 bis 30 Stunden und sind von der eingestellten Trocknungstemperatur abhängig, wobei eine höhere Temperatur die Trocknungszeit verkürzt. Die Trocknung kann durch Anwenden eines Unterdrucks weiter beschleunigt werden.

Im Anschluss an die Trocknung werden die Formkörper durch Kalzination zum fertigen Träger umgewandelt. Die Kalzinationstemperatur liegt im Allgemeinen im Bereich von 900 - 1150 °C, vorzugsweise im Bereich von 1000 - 1120 °C, besonders bevorzugt im Bereich von 1050 - 1100 °C. Die Kalzinationsdauer liegt im Allgemeinen zwischen 0,5 und 5 Stunden, vorzugsweise zwischen 1 und 4 Stunden, besonders bevorzugt zwischen 1,5 und 3 Stunden. Die Kalzination erfolgt in einem üblichen Ofen, beispielsweise in einem Drehofen, in einem Tunnelofen, in einem Bandkalzinierer oder in einem Kammerofen. Die Kalzination kann sich ohne zwischenzeitliche Abkühlung der Formkörper direkt an die Trocknung anschließen.

Die so erhaltenen erfindungsgemäßen Katalysatoren weisen eine spezifische Oberfläche (BET, Brunauer - Emmet - Teller, bestimmt gemäß DIN 66131 durch Stickstoffadsorption bei 77 K) von 20 - 250 m²/g, vorzugsweise 50 - 150 m²/g, insbesondere 60 - 90 m²/g, auf. Die Oberfläche kann durch bekannte Methoden, insbesondere Verwendung feinteiliger oder gröberer Ausgangsstoffe, Kalzinationsdauer und Kalzinationstemperatur, variiert werden. Wie die BET-Oberfläche kann auch das Porenvolumen auf bekannte Weise variiert werden, im Allgemeinen liegt es, mittels Quecksilberporosymmetrie bestimmt, in einem Bereich von 0,3 - 1,0 ml/g, vorzugsweise in einem Bereich von 0,4 - 0,9 ml/g, besonders bevorzugt 0,5 - 0,8 ml/g.

Nach der Kalzination werden auf dem so hergestellten Träger die Aktivmasse und gegebenenfalls weitere Zusatzstoffe abgeschieden.

Der Träger des erfindungsgemäßen Katalysators ist vorzugsweise durch folgendes Röntgenbeugungsdiagramm charakterisiert:

| Netzebenenabstand | Winkel | Intensität |
|---|---|---|
| Angstrom [Å] | 2-Theta [°] | [%] |
| d = 4,552 | 19, 483 | 5 - 15 |
| d = 2,857 | 31,278 | 35 - 50 |
| d = 2,730 | 32,775 | 65 - 80 |
| d = 2,449 | 36,671 | 45 - 55 |
| d = 2,317 | 38,842 | 35 - 45 |
| d = 2,260 | 39,861 | 35 - 45 |
| d = 2,022 | 44,790 | 45 - 65 |
| d = 1,910 | 47,570 | 30 - 40 |
| d = 1,798 | 50,720 | 10 - 25 |
| d = 1,543 | 59,915 | 25 - 35 |
| d = 1,511 | 61,307 | 0 - 35 |
| d = 1,489 | 62,289 | 20 - 30 |
| d = 1,455 | 63,926 | 25 - 35 |
| d = 1,387 | 67,446 | 100 |

Dieses Röntgenbeugungsdiagramm wird wie in der EP 0 992 284 A2 auf Seite 9, Zeilen 6 bis 9 beschrieben, bestimmt.

Röntgenbeugungsdiagramme sind charakteristisch für die spezifische Struktur des untersuchten Materials. Die Struktur des erfindungsgemäßen Katalysators ist durch das Auftreten der oben genannten Reflexe hinreichend definiert. Zusätzlich zu den oben angegebenen kennzeichnenden Reflexen können im Röntgenbeugungsdiagramm ein oder mehrere Reflexe in beliebiger Intensität für die Netzebenenabstände 3,48; 2,55; 2,38; 2,09; 1,78; 1,74; 1,62; 1,60; 1,57; 1,42; 1,40 und/oder 1,37 alle in der Einheit [Å], auftreten.

Weiterhin können im Röntgenbeugungsdiagramm des erfindungsgemäßen Katalysators noch beliebige weitere Reflexe auftreten.

Auf den so erhaltenen Träger des erfindungsgemäßen Katalysators können die Aktivmasse und gegebenenfalls weitere Zusatzstoffe abgeschieden werden.

Die auf den Träger abzuscheidenden Metalle, Zusatz- und/oder Dotierstoffe können mit jedem bekannten Verfahren auf den Träger aufgebracht werden, beispielsweise durch Beschichtung aus der Gasphase (*chemical* oder *physical vapor deposition*) oder Tränkung des Trägermaterials in einer Lösung, welche die abzuscheidenen Substanzen und/oder Verbindungen enthält.

Die bevorzugte Methode ist die Tränkung mit einer Lösung der abzuscheidenden Substanzen und/oder Verbindungen, die sich im Zuge der weiteren Katalysatorherstellung in die abzuscheidenden Substanzen umwandeln. Die abzuscheidenden Substanzen können einzeln und/oder in Teilmengen in mehreren Verfahrensschritten oder gemeinsam und vollständig in einem Verfahrensschritt abgeschieden werden. Bevorzugt ist die gemeinsame Abscheidung in einer Tränkstufe. Im Anschluss an die Tränkung oder nach den einzelnen Tränkstufen wird der geträgerte Katalysator getrocknet und durch Kalzinierung sowie gegebenenfalls andere bekannte Nachbehandlungsmethoden, beispielsweise Aktivierung und anschließende oberflächliche Passivierung, zum einsatzbereiten Katalysator umgewandelt.

Tränkverfahren zur Abscheidung von Aktivkomponenten, Zusatzstoffen und/oder Dotierstoffen auf einem Träger sind bekannt. Im Allgemeinen wird der Träger mit einer Lösung von Salzen der abzuscheidenden Komponenten getränkt, wobei das Volumen der Lösung so bemessen wird, dass die Lösung praktisch vollständig vom Porenvolumen des Trägers aufgenommen wird ("incipient wetness"-Methode). Die Konzentration der Salze in der Lösung wird so bemessen, dass nach Tränkung und Umwandlung des geträgerten Katalysators zum fertigen Katalysator die abzuscheidenden Komponenten in der gewünschten Konzentration auf dem Katalysator vorliegen. Die Salze werden so gewählt, dass sie keine bei der Katalysatorherstellung oder dessen späterer Verwendung störenden Rückstände hinterlassen. Zumeist werden Nitrate oder Ammoniumsalze verwendet.

Grundsätzlich eignen sich alle dem Fachmann bekannten Tränkverfahren zur Herstellung des erfindungsgemäßen Katalysators.

Die Herstellung des erfindungsgemäßen Katalysators erfolgt jedoch vorzugsweise unter einstufiger Tränkung des Trägers nach der incipient-wetness-Methode einer salpetersauren Lösung der Nitrate der abzuscheidenden Metalle.

In einer besonders bevorzugten Ausführungsform wird eine Tränklösung verwendet, die Palladiumnitrat und -nitrit nebeneinander enthält.

Darüber hinaus liegt in der Tränklösung noch das Metall der IB. Gruppe des Periodensystems der Elemente, vorzugsweise Silbernitrat, vor.

Im Allgemeinen liegt der pH-Wert der Tränklösung bei höchstens 5, vorzugsweise bei höchstens 2, besonders bevorzugt bei höchstens 1, insbesondere bei höchstens 0,5. Die Untergrenze des pH-Wertes liegt im Allgemeinen bei 0,2, vorzugsweise bei 0,3, besonders bevorzugt bei 0,5. Ein besonders bevorzugter pH-Bereich liegt bei 0,3 bis 0,5.

Nach der Tränkung wird der getränkte Träger in üblicher Weise getrocknet, im Allgemeinen bei einer Temperatur oberhalb von 60°C, vorzugsweise oberhalb von 80 °C, besonders bevorzugt oberhalb von 100°C, insbesondere bei einer Temperatur im Bereich von 120 - 300 °C. Die Trocknung wird dabei fortgesetzt, bis im getränkten Katalysator vorhandenes Wasser im Wesentlichen vollständig entwichen ist, was im Allgemeinen nach einigen Stunden der Fall ist. Übliche Trocknungszeiten liegen im Bereich von 1 - 30 Stunden und sind von der eingestellten Trocknungstemperatur abhängig, wobei eine höhere Trocknungstemperatur die Trocknungszeit verkürzt. Die Trocknung kann durch Anwenden eines Unterdrucks weiter beschleunigt werden.

In einer besonders bevorzugten Ausführungsform des erfindungsgemäßen Verfahrens erfolgt die Trocknung des getränkten Katalysators unter gleichzeitiger Bewegung des getränkten Trägermaterials, beispielsweise in einem Drehrohr-Ofen.

In einer besonderen Ausführungsform der vorliegenden Erfindung wird der zur Trocknung verwendete Luftstrom im Gegenstrom durch das Drehrohr geführt.

Im Anschluss an die Trocknung wird in üblicher Weise durch Kalzinierung der Katalysator hergestellt. Diese Kalzinierung dient im Wesentlichen der Umwandlung der aufgetränkten Salze in die abzuscheidenden Komponenten oder Vorläufer solcher Komponenten und unterscheidet sich insofern von der zuvor beschriebenen Kalzinierung, die der Herstellung des Trägermaterials und der Trägerstruktur dient. Im Fall der Auftränkung von Metallnitraten werden bei dieser Kalzinierung im Wesentlichen die Nitrate in Metalle und/oder Metalloxide, die im Katalysator verbleiben, und in nitrose Gase, die entweichen, zersetzt.

Die Kalzinationstemperatur liegt im Allgemeinen bei 200 - 900 °C, vorzugsweise 280 - 800 °C, besonders bevorzugt 300 - 700 °C. Die Kalzinationsdauer liegt im Allgemeinen zwischen 0,5 und 20 Stunden, vorzugsweise zwischen 0,5 und 10 Stunden, besonders bevorzugt zwischen 0,5 und 5 Stunden. Die Kalzination erfolgt in einem üblichen Ofen, beispielsweise in einem Drehrohr-Ofen, in einem Bandkalzinierer oder in einem Kammerofen. Die Kalzination kann sich ohne zwischenzeitliche Abkühlung des geträgerten und getrockneten Katalysators direkt an die Trocknung anschließen.

In einer besonders bevorzugten Ausführungsform des erfindungsgemäßen Verfahrens erfolgt die Trocknung und die Kalzinierung des Katalysators in einem Drehrohrofen kombiniert.

Nach der Kalzination ist der Katalysator prinzipiell einsatzbereit. Falls erforderlich oder gewünscht, wird er vor dem Einbau in den Hydrierreaktor in bekannter Weise durch Vorreduzierung aktiviert und gegebenenfalls auch wieder oberflächlich passiviert.

In der Regel erfolgt die Reduktion des Hydrierkatalysators jedoch meistens erst im Hydrierreaktor selbst. Dies geschieht nach einer dem Fachmann bekannten Weise durch zunächst erfolgende Inertisierung mit Stickstoff oder einem anderen Inertgas. Die Reduktion wird, mit einem Wasserstoff-haltigen Gas als reine Gasphase oder unter Inert-Kreislauf durchgeführt. Die Temperatur, bei der diese Vorreduktion durchgeführt wird, beträgt im Allgemeinen 5 - 200 °C, vorzugsweise 20 - 150 °C.

Auch eine Regenerierung des erfindungsgemäßen Katalysators ist außer- oder innerhalb des Hydrierreaktors bei Temperaturen von 15 bis 500°C möglich.

Die Hydrierung des Austrages aus dem Steamcracker an dem zuvor beschriebenen Katalysator erfolgt vorzugsweise bei einem Druck von 6 x 10⁵ bis 51 x 10⁵ Pa (5 bis 50 barg). Die Eintrittstemperatur beträgt vorzugsweise 20 bis 100°C, wobei die Temperaturerhöhung vorzugsweise 10 bis 60 °C beträgt. Die Frischfeed-Belastung (whsv) beträgt vorzugsweise 0,5 bis 15 kg/lh. Das Verhältnis Kreislaufstrom zu Frischfeed beträgt vorzugsweise 2 bis 20 und die Leerraumgeschwindigkeit beträgt vorzugsweise 20 bis 400 m/h. Das Verhältnis von Wasserstoff zu Butadien beträgt vorzugsweise 1 bis 1,5.

Durch diese Hydrierung wird vorzugsweise ein C4-Olefin-Gemisch mit mit einem Gehalt an 1,3-Butadien von 100 bis 500 ppm, vorzugsweise 110 bis 400 ppm, besonders bevorzugt 120 bis 300 ppm, und einem Gehalt an kumulierten Dienen wie Propadien, 1,2-Butadien, 1,2-Pentadien oder 2,3-Pentadien von weniger als 10 ppm, vorzugsweise von 1 bis 10 ppm, besonders bevorzugt von 2 bis 10 ppm, erhalten.

Der 1-Buten-Gehalt im hydrierten C4-Strom beträgt vorzugsweise 30 %, besonders bevorzugt 40 %, insbesondere 50 % (nach Isobuten-Entfernung, Rest Isobuten: vorzugsweise 0,5 bis 4 %, besonders bevorzugt 1 bis 3 %), während das Verhältnis von 1-Buten zu 2-Buten vorzugsweise 1,2 bis 2,0, besonders bevorzugt 1,3 bis 1,6 beträgt.

In einer besonders bevorzugten Ausführungsform wird das erfindungsgemäße Verfahren mehrstufig, besonders bevorzugt dreistufig durchgeführt.

In diesem Ausführungsform findet die erste Hydrierstufe an dem zuvor beschriebenen erfindungsgemäßen Katalysator derart statt, dass der zu hydrierende Strom aus dem Steamcracker als im Wesentlichen flüssige Phase, die auch Wasserstoff im Rahmen dessen Löslichkeit enthalten kann, mit einer im Wesentlichen gasförmigen Wasserstoff-haltigen Phase umgesetzt wird. Alternativ kann die Hydrierung auch in im Wesentlichen nur einer flüssigen Phase erfolgen, so dass der Wasserstoff komplett eingelöst in der flüssigen Phase vorliegt.

Die Eintrittstemperatur beträgt dabei vorzugsweise 20 bis 100°C, besonders bevorzugt 30 bis 90 °C, insbesondere 30 bis 80 °C. Die Hydrierung erfolgt unter einer Temperatureerhöhung von vorzugsweise 10 bis 60 °C, besonders bevorzugt 20 bis 50 °C, insbesondere 25 bis 45 °C.

Der Druck während der ersten Hydrierung beträgt vorzugsweise 6 x 10⁵ bis 51 x 10⁵ Pa (5 bis 50 barg), besonders bevorzugt 6 x 10⁵ bis 31 x 10⁵ Pa (5 bis 30 barg), insbesondere 11 x 10⁵ bis 31 x 10⁵ Pa (10 bis 30 barg).

Die Frischfeed-Belastung (whsv) beträgt in der ersten Hydrierstufe vorzugsweise 0,5 bis 15 kg/lh, besonders bevorzugt 1 bis 10 kg/lh, insbesondere 1,5 bis 8 kg/lh.

Die erste Hydrierstufe wird vorzugsweise so durchgeführt, dass die Leerraumgeschwindigkeit während der Hydrierung vorzugsweise 20 bis 400 m/h beträgt.

Das Verhältnis von Wasserstoff zu Butadien beträgt in der ersten Hydrierstufe vorzugsweise 0,7 bis 1,5, besonders bevorzugt 0,8 bis 1,2, insbesondere 0,9 bis 1,0, wobei der Austrittsgehalt an Butadien vorzugsweise 0,05 bis 10 Gew.-%, besonders bevorzugt 2 bis 6 Gew.-%, insbesondere 3 bis 5 Gew.-%, beträgt.

Das Verhältnis Kreislaufstrom zu Frischfeed beträgt vorzugsweise 2 bis 20, besonders bevorzugt 2 bis 15, insbesondere 5 bis 12.

In der ersten Hydrierstufe kann der Gehalt an inerten Verbindungen in dem zur Hydrierung verwendeten Wasserstoff vorzugsweise 0 bis 30 Vol.-%, besonders bevorzugt 0 bis 15 Vol.-%, betragen.

Die Hydrierung der zweiten Verfahrensstufe kann grundsätzlich an jedem geeigneten Katalysator durchgeführt werden.

Bei dem Hydrierkatalysator für die zweite Hydrierstufe kann es sich beispielsweise um einen Katalysator, der mindestens ein Element der VIII. Gruppe des Periodensystems der Elemente umfasst, handeln, wobei eines dieser Elemente vorzugsweise Palladium ist. Der Gehalt an diesem Element in dem Katalysator, bezogen auf den Katalysator, beträgt vorzugsweise 0,05 bis 2 Gew.-%, besonders bevorzugt 0,1 bis 0,3 Gew.-%.

Der Katalysator enthält vorzugsweise mindestens einen Promotor, wobei es bevorzugt ist, wenn es sich hierbei um ein Element der IB. Gruppe des Periodensystems der Elemente, insbesondere um Silber handelt. Die Menge an Promotor beträgt vorzugsweise 0,01 bis 1 Gew.-%, besonders bevorzugt 0,05 bis 0,5 Gew.-%.

Es ist besonders bevorzugt, wenn in dem Katalysator das integrale Palladium/Silber-Atomverhältnis 0,1 bis 10, besonders bevorzugt 0,5 bis 5, beträgt.

Der Katalysator umfasst vorzugsweise einen oxidischen Träger, wobei als Trägermaterial Aluminiumoxid (Al₂O₃) bevorzugt ist. Die BET-Oberfläche des Trägermaterials beträgt vorzugsweise 1 bis 250 m²/g, besonders bevorzugt 30 bis 150 m²/g, insbesondere 60 bis 90 m²/g.

Die Hydrierung in der zweiten Verfahrensstufe wird vorzugsweise wie folgt durchgeführt:
Die Hydrierung in der zweiten Verfahrensstufe kann mit einer im Wesentlichen flüssigen C4-Olefin-Gemischphase, die auch Wasserstoff im Rahmen dessen Löslichkeit enthalten kann, mit einer im Wesentlichen gasförmigen Wasserstoff-haltigen Phase durchgeführt werden. Alternativ kann die Hydrierung auch in im Wesentlichen nur einer flüssigen Phase erfolgen, so dass der Wasserstoff komplett eingelöst in der flüssigen Phase vorliegt.

Der Druck in der zweiten Verfahrensstufe beträgt vorzugsweise 6 x 10⁵ bis 51 x 10⁵ Pa (5 bis 50 barg), besonders bevorzugt 6 x 10⁵ bis 31 x 10⁵ Pa (5 bis 30 barg), insbesondere 11 x 10⁵ bis 31 x 10⁵ Pa (10 bis 30 barg).

Die Eintrittstemperatur in die zweite Hydrierstufe beträgt vorzugsweise 20 bis 100 °C, besonders bevorzugt 30 bis 90 °C, insbesondere 40 bis 90°C. Dabei wird die Hydrierung unter einer Temperaturerhöhung von vorzugsweise 10 bis 60 °C, besonders bevorzugt 5 bis 20 °C, insbesondere 5 bis 15°C, durchgeführt.

Die Frischfeed-Belastung (whsv) beträgt vorzugsweise 0,5 bis 15 kg/lh, besonders bevorzugt 1 bis 12 kg/lh, insbesondere 2 bis 10 kg/lh.

Die Hydrierung kann im Kreislauf oder im geraden Durchgang gefahren werden. Das Verhältnis Kreislaufstrom zu Frischfeed beträgt dabei vorzugsweise 0 bis 20, besonders bevorzugt 0 bis 10, insbesondere 0 bis 7.

Die Leerraumgeschwindigkeit in der zweiten Hydrierstufe beträgt vorzugsweise 20 bis 400 m/h.

Das Verhältnis von Wasserstoff zu Butadien beträgt in der zweiten Hydrierstufe vorzugsweise 1 bis 3, besonders bevorzugt 1 bis 2, insbesondere 1 bis 1,3.

Wenn das erfindungsgemäße Verfahren mindestens dreistufig durchgeführt wird, so ergibt sich ein Austrittsgehalt an 1,3-Butadien nach der zweiten Hydrierstufe von vorzugsweise 0,005 bis 0,05 Gew.-%, besonders bevorzugt 0,01 bis 0,5 Gew.-%, insbesondere 0,1 bis 0,2 Gew.-%. Die gesamte Summe an kumulierten Dienen beträgt dann vorzugsweise 0 bis 100 Gew.-ppm, besonders bevorzugt 0 bis 10 Gew.-ppm.

Wenn das erfindungsgemäße Verfahren zweistufig durchgeführt wird, so ergibt sich ein C4-Olefin-Gemisch mit einem Gehalt an 1,3-Butadien von 100 bis 500 ppm, vorzugsweise 110 bis 400 ppm, besonders bevorzugt 120 bis 300 ppm, und einem Gehalt an kumulierten Dienen wie Propadien, 1,2-Butadien, 1,2-Pentadien oder 2,3-Pentadien von weniger als 10 ppm, vorzugsweise von 1 bis 10 ppm, besonders bevorzugt von 2 bis 10 ppm.

Die Reaktionsführung in der zweiten Hydrierstufe erfolgt - analog zur ersten Hydrierstufe - auf dem Fachmann an sich bekannte Weise, z. B. adiabatisch oder mit Siedekühlung.

Der Gehalt an inerten Verbindungen in dem Wasserstoff, der in der zweiten Hydrierstufe verwendet wird, beträgt vorzugsweise 0 bis 30 Vol.-%, besonders bevorzugt 0 bis 15 Vol.-%.

Der Gehalt an 1-Buten im hydrierten C4-Gemisch beträgt nach i-Buten-Entfernung vorzugsweise 30 %, besonders bevorzugt 40 %, insbesondere 50 %, wobei das Verhältnis von 1-Buten zu 2-Buten vorzugsweise 1,2 bis 2,0, besonders bevorzugt 1,3 bis 1,6 beträgt.

Das erfindungsgemäße Verfahren kann darüber hinaus weitere Hydrierstufen oder Methoden zur Bestimmung des 1,2-Diengehalts umfassen.

Falls das erfindungsgemäße Verfahren eine dritte Hydrierstufe (Feinhydrierung) umfasst, so wird diese Hydrierstufe vorzugsweise bei den folgenden Bedingungen durchgeführt:
Wenn in dem erfindungsgemäßen Verfahren eine weitere dritte Hydrierstufe durchgeführt wird, so kann diese mit jedem beliebigen geeigneten Katalysator durchgeführt werden, wobei auch der in der ersten Hydrierstufe verwendete Katalysator verwendet werden kann.

Bei dem Hydrierkatalysator für diese optionale dritte Hydrierstufe kann es sich beispielsweise um einen Katalysator, der mindestens ein Element der VIII. Gruppe des Periodensystems der Elemente umfasst, handeln, wobei eines dieser Elemente vorzugsweise Palladium ist. Der Gehalt an diesem Element in dem Katalysator, bezogen auf den Katalysator, beträgt vorzugsweise 0,05 bis 2 Gew.-%, besonders bevorzugt 0,1 bis 0,3 Gew.-%.

Der Katalysator enthält vorzugsweise mindestens einen Promotor, wobei es bevorzugt ist, wenn es sich hierbei um ein Element der IB. Gruppe des Periodensystems der Elemente, insbesondere um Silber handelt. Die Menge an Promotor beträgt vorzugsweise 0,01 bis 1 Gew.-%, besonders bevorzugt 0,05 bis 0,5 Gew.-%.

Es ist besonders bevorzugt, wenn in dem Katalysator das integrale Palladium/Silber-Atomverhältnis 0,1 bis 10, besonders bevorzugt 0,5 bis 5, beträgt.

Der Katalysator umfasst vorzugsweise einen oxidischen Träger, wobei als Trägermaterial Aluminiumoxid (Al₂O₃) bevorzugt ist. Die BET-Oberfläche des Trägermaterials beträgt vorzugsweise 1 bis 250 m²/g, besonders bevorzugt 30 bis 150 m²/g, insbesondere 60 bis 90 m²/g.

Die Hydrierung in der dritten Verfahrensstufe kann mit einer im Wesentlichen flüssigen C4-Olefin-Gemischphase, die auch Wasserstoff im Rahmen dessen Löslichkeit enthalten kann, mit einer im Wesentlichen gasförmigen Wasserstoff-haltigen Phase durchgeführt werden. Alternativ kann die Hydrierung auch in im Wesentlichen nur einer flüssigen Phase erfolgen, so dass der Wasserstoff komplett eingelöst in der flüssigen Phase vorliegt.

Der Druck beträgt dabei vorzugsweise 6 x 10⁵ bis 31 x 10⁵ Pa (5 bis 30 barg), besonders bevorzugt 11 x 10⁵ bis 31 x 10⁵ Pa (10 bis 30 barg). Die Eintrittstemperatur in die Hydrierung der dritten Verfahrensstufe beträgt vorzugsweise 30 bis 90 °C, besonders bevorzugt 40 bis 85 °C. Dabei wird eine Temperaturerhöhung von vorzugsweise 0 bis 20 °C, besonders bevorzugt 0 bis 10 °C, eingestellt.

Die Frischfeed-Belastung (whsv) beträgt vorzugsweise 1 bis 12 kg/lh, besonders bevorzugt 3 bis 10 kg/lh.

Das Verhältnis von Wasserstoff zu Butadien beträgt vorzugsweise 1 bis 10, besonders bevorzugt 1 bis 8.

Der Gehalt an inerten Verbindungen in dem zur Hydrierung verwendeten Wasserstoff kann 0 bis 30 Vol.-%, insbesondere 0 bis 15 Vol.-%, betragen.

Der 1-Buten-Gehalt im hydrierten C4-Strom beträgt vorzugsweise 30 %, besonders bevorzugt 40 %, insbesondere 50 % (nach Isobuten-Entfernung, Rest Isobuten: vorzugsweise 0,5 bis 4 %, besonders bevorzugt 1 bis 3 %), während das Verhältnis von 1-Buten zu 2-Buten vorzugsweise 1,2 bis 2,0, besonders bevorzugt 1,3 bis 1,6 beträgt.

Die Reaktionsführung kann auf übliche Art und Weise, die dem Fachmann bekannt ist, durchgeführt werden, beispielsweise adiabatisch oder mit Siedekühlung.

Der Butadienaustrittsgehalt nach dieser dritten Hydrierstufe beträgt vorzugsweise 0,07 bis 0,05 Gew.%, besonders bevorzugt 0,01 bis 0,02 Gew.-%.

Die gesamte Summe an Dienen, die nach der dritten Hydrierstufe im Strom verbleiben beträgt vorzugsweise 0 bis 10 Gew.-ppm.

In einer bevorzugten Ausführungsform der vorliegenden Erfindung eignet sich das erfindungsgemäße Verfahren zur Herstellung des erfindungsgemäßen C4-Olefin-Stroms mit einem Gehalt an 1,3-Butadien von 100 bis 500 ppm, vorzugsweise 110 bis 400 ppm. Besonders bevorzugt 120 bis 300 ppm, und einem Gehalt an kumulierten 1,2-Dienen wie Propadien, 1,2-Butadien, 1,2-Pentadien oder 2,3-Pentadien von weniger als 10 ppm, vorzugsweise von 1 bis 10 ppm, besonders bevorzugt von 2 bis 10 ppm. Weiterer Gegenstand der vorliegenden Erfindung sind die durch das zuvor beschriebene Selektivhydrierverfahren erhältlichen C4-Olefin-Gemische.

Weiterer Gegenstand der vorliegenden Erfindung ist ein Verfahren zur Herstellung eines 2-Penten und/oder 3-Hexen enthaltenden Olefin-Gemisches durch Umsetzung des zuvor beschriebenen C₄-Olefin-Gemisches an einem Metathese-Katalysator. Im Sinne der vorliegenden Erfindung werden unter dem Begriff "2-Penten und/oder 3-Hexen enthaltendes Olefin-Gemisch" Gemische, die 2-Penten und/oder 3-Hexen, sowie reines 2-Penten und reines 3-Hexen verstanden.

Die Metathese kann beispielsweise wie in WO 00/39058 oder DE-A-100 13 253 beschrieben durchgeführt werden.

Die Olefinmetathese (Disproportionierung) beschreibt in ihrer einfachsten Form die reversible, metallkatalysierte Umalkylidenierung von Olefinen durch Bruch oder Neuformierung von C=C-Doppelbindungen gemäß nachfolgender Gleichung:

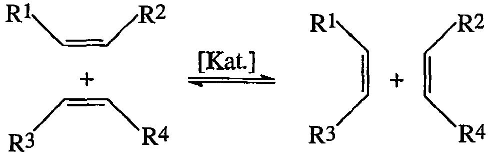

Im speziellen Fall der Metathese von acyclischen Olefinen unterscheidet man zwischen Selbstmetathese, bei der ein Olefin in ein Gemisch zweier Olefine unterschiedlicher molarer Masse übergeht (beispielsweise: Propen → Ethen + 2-Buten), und Kreuz- oder Co-Metathese, die eine Reaktion zweier unterschiedlicher Olefine beschreibt (Propen + 1-Buten → Ethen + 2-Penten). Ist einer der Reaktionspartner Ethen, so spricht man im Allgemeinen von einer Ethenolyse.

Als Metathesekatalysatoren eignen sich prinzipiell homogene und heterogene Übergangsmetall-Verbindungen, insbesondere die der VI. bis VIII.-Nebengruppe des Periodensystems der Elemente sowie homogene und heterogene Katalysatorsysteme, in denen diese Verbindungen enthalten sind.

Im Rahmen der vorliegenden Erfindung sind im Allgemeinen alle Metathese-Verfahren einsetzbar, die von C₄-Strömen ausgehen.

So beschreibt die DE 199 32 060 A1 ein Verfahren zur Herstellung von C₅-/C₆-Olefinen durch Umsetzung eines Ausgangsstroms, der 1-Buten, 2-Buten und Isobuten enthält, zu einem Gemisch aus C₂₋₆-Olefinen. Dabei wird aus Butenen insbesondere Propen gewonnen. Zusätzlich werden Hexen und Methylpenten als Produkte ausgeschleust. In der Metathese wird kein Ethen zudosiert. Gegebenenfalls wird in der Metathese gebildetes Ethen in den Reaktor zurückgeführt.

Das im Rahmen der vorlegenden Erfindung jedoch bevorzugte Metathese-Verfahren ist die Herstellung von 2-Penten und/oder 3-Hexen enthaltenden Strömen aus dem zuvor beschriebenen, erfindungsgemäß behandelten olefinischen C₄-Kohlenwasserstoffe enthaltenden Raffinat-II-Ausgangsstrom. Dieses Verfahren ist in einer ersten Ausführungsform dadurch gekennzeichnet, dass
a) in Gegenwart eines Metathesekatalysators, der mindestens eine Verbindung eines Metalls der VIb., VIIb. oder VIII. Nebengruppe des Periodensystems der Elemente enthält, eine Metathesereaktion durchgeführt wird, im Rahmen derer im Ausgangsstrom enthaltene Butene mit Ethen zu einem Ethen, Propen, Butene, 2-Penten, 3-Hexen und Butane enthaltenden Gemisch umgesetzt werden, wobei bezogen auf die Butene bis 0,6 Moläquivalente Ethen eingesetzt werden können,
b) der so erhaltene Austragsstrom zunächst destillativ getrennt wird in gegebenenfalls eine C₂-C₃-Olefine enthaltende Leichtsiederfraktion A sowie in eine C₄-C₆-Olefine und Butane enthaltende Schwersiederfraktion,
c) die aus b) gegebenenfalls erhaltene Leichtsiederfraktion **A** anschließend destillativ in eine Ethen enthaltende Fraktion und eine Propen enthaltende Fraktion getrennt wird, wobei die Ethen enthaltende Fraktion in den Verfahrensschritt a) zurückgeführt wird und die Propen enthaltende Fraktion als Produkt ausgeschleust wird,
d) die aus b) erhaltene Schwersiederfraktion anschließend destillativ in eine Butene und Butane enthaltende Leichtsiederfraktion **B,** eine 2-Penten enthaltende Mittelsiederfraktion **C** und in eine 3-Hexen enthaltende Schwersiederfraktion **D** getrennt wird,
e) wobei die Fraktionen **B** und gegebenenfalls **C** komplett oder teilweise in den Verfahrensschritt a) zurückgeführt werden und die Fraktion **D** und gegebenenfalls **C** als Produkt ausgeschleust werden.

In einer zweiten Ausführungsform sind folgende Verfahrensschritte vorgesehen:
a) in Gegenwart eines Metathesekatalysators, der mindestens eine Verbindung eines Metalls der VIb., VIIb. oder VIII. Nebengruppe des Periodensystems der Elemente enthält, eine Metathesereaktion durchgeführt wird, im Rahmen derer im Ausgangsstrom enthaltene Butene mit Ethen zu einem Ethen, Propen, Butene, 2-Pentan, 3-Hexen und Butane enthaltenden Gemisch umgesetzt werden, wobei bezogen auf die Butene bis 0,6 Moläquivalente Ethen eingesetzt werden können,
b) der so erhaltene Ausgangsstrom zunächst destillativ getrennt wird in eine C₂-Leichtsiederfraktion, die über den Kopf einer Destillationskolonne entfernt wird, eine C₃-Leichtsiederfraktion, die anschließend ebenfalls über Kopf der Destillationskolonne entfernt wird, unter Erhalt einer C₄-C₆-Olefine und Butane enthaltende Schwersiederfraktion;
c) Rückführung der in Verfahrensschritt (2) erhaltenen C2-Leichtiederfraktion in den Verfahrensschritt (1) und Ausschleusung der C3-Leichsiederfraktion als Produkt;
d) die aus b) erhaltene Schwersiederfraktion anschließend destillativ in eine Butene und Butane enthaltende Leichtsiederfraktion **B,** eine 2-Penten enthaltende Mittelsiederfraktion **C** und in eine 3-Hexen enthaltende Schwersiederfraktion **D** getrennt wird,
e) wobei die Fraktionen **B** und gegebenenfalls **C** komplett oder teilweise in den Verfahrensschritt a) zurückgeführt werden und die Fraktion **D** und gegebenenfalls **C** als Produkt ausgeschleust werden.

In der zweiten Ausführungsform ist somit im Unterschied zur ersten Ausführungsform vorgesehen, dass anstelle der Verfahrensschritte b) und c) aus der in Verfahrensschritt a) erhaltenen Ausgangsstrom zunächst über Kopf einer Destillationskolonne eine C2-Fraktion (Leichtsiederfraktion A1) und anschließend über Kopf der Destillationskolonne eine C3-Fraktion (Leichtsiederfraktion A2) abgetrennt wird unter Erhalt einer C₄-C₆-Olefine und Butane enthaltenden Schwersiederfraktion, die dann in Verfahrensschritt d) weiterverarbeitet wird.

Die einzelnen Ströme und Fraktionen können die genannten Verbindungen enthalten oder aus ihnen bestehen. Im Fall, dass sie aus den Strömen oder Verbindungen bestehen, ist die Gegenwart kleinerer Mengen anderer Kohlenwasserstoffe nicht ausgeschlossen.

Dabei wird in einstufiger Reaktionsführung in einer Metathesereaktion die erfindungsgemäße aus C₄-Olefinen, vorzugsweise n-Butenen und Butanen bestehende Fraktion gegebenenfalls mit variablen Mengen Ethen an einem homogenen oder vorzugsweise heterogenen Metathesekatalysator zu einem Produktgemisch aus (inerten) Butanen, nicht umgesetztem 1-Buten, 2-Buten sowie den Metatheseprodukten Ethen, Propen, 2-Penten und 3-Hexen umgesetzt. Die gewünschten Produkte 2-Penten und/oder 3-Hexen werden ausgeschleust, und die verbleibenden Produkte und nicht umgesetzten Verbindungen werden in die Metathese ganz oder teilweise zurückgeführt. Vorzugsweise werden sie möglichst vollständig zurückgeführt, wobei nur geringe Mengen ausgeschleust werden, um eine Aufpegelung zu vermeiden. Idealerweise kommt es zu keiner Aufpegelung und alle Verbindungen außer 3-Hexen werden in die Metathese zurückgeführt.

Erfindungsgemäß werden, bezogen auf die Butene im erfindungsgemäßen C₄-Feedstrom, bis 0,6, vorzugsweise bis 0,5 Moläquivalente Ethen eingesetzt. Damit werden im Vergleich zum Stand der Technik nur geringe Ethenmengen eingesetzt.

Wenn auf die Zuführung von zusätzlichem Ethen verzichtet wird, werden im Verfahren nur bis zu maximal etwa 1,5 %, bezogen auf die Umsetzungsprodukte, an Ethen gebildet, das zurückgeführt wird, siehe DE 199 32 060 A1. Es können auch erfindungsgemäß größere Ethenmengen eingesetzt werden, wobei die eingesetzten Mengen wesentlich geringer sind als in den bekannten Verfahren zur Herstellung von Propen.

Zudem werden erfindungsgemäß maximal mögliche Mengen an im Reaktoraustrag enthaltenen C₄-Produkten und gegebenenfalls C₅-Produkten zurückgeführt. Dies betrifft insbesondere die Rückführung von nicht umgesetztem 1-Buten und 2-Buten sowie gegebenenfalls gebildetem 2-Penten.

Sofern im C₄-Feedstrom noch geringe Mengen an Isobuten enthalten sind, können auch geringe Mengen verzweigter Kohlenwasserstoffe gebildet werden.

Die Menge an möglicherweise zusätzlich gebildeten verzweigten C₅- und C₆-Kohlenwasserstoffen im Metatheseaustrag ist abhängig vom Isobuten-Gehalt im C₄-Feed und wird vorzugsweise möglichst gering (< 3 %) gehalten.

Um das erfindungsgemäße Verfahren in mehreren Variationen näher zu erläutern, wird die im Metathesereaktor stattfindende Umsetzung in drei wichtige Einzelreaktionen unterteilt:
**1. Kreuzmetathese von 1-Buten mit 2-Buten** 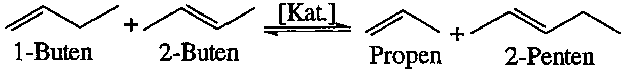
**2. Selbstmetathese von 1-Buten** 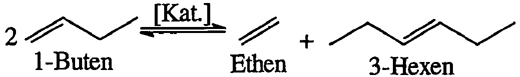
**3. Gegebenenfalls Ethenolyse von 2-Buten**

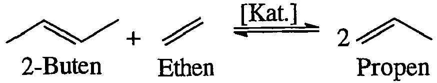

In Abhängigkeit vom jeweiligen Bedarf an den Zielprodukten Propen und 3-Hexen (die Bezeichnung 3-Hexen beinhaltet im Rahmen der vorliegenden Erfindung unter anderem gegebenenfalls gebildete Isomere) bzw. 2-Penten kann die äußere Massenbilanz des Verfahrens gezielt durch variablen Einsatz von Ethen und durch Verschiebung des Gleichgewichts durch Rückführung bestimmter Teilströme beeinflußt werden. So wird beispielsweise die 3-Hexenausbeute dadurch erhöht, dass durch Rückführung von 2-Penten in den Metatheseschritt die Kreuzmetathese von 1-Buten mit 2-Buten unterdrückt wird, so dass hier kein oder möglichst wenig 1-Buten verbraucht wird. Bei der dann bevorzugt ablaufenden Selbstmetathese von 1-Buten zu 3-Hexen wird zusätzlich Ethen gebildet, welches in einer Folgereaktion mit 2-Buten zum Wertprodukt Propen reagiert.

Der Butengehalt der im Verfahren eingesetzten erfindungsgemäßen C₄-Fraktion beträgt 1 bis 100 Gew.-%, vorzugsweise 60 bis 90 Gew.-%. Der Butengehalt bezieht sich dabei auf 1-Buten, 2-Buten und Isobuten.

Das erfindungsgemäß eingesetzte zuvor beschriebene C₄-Olefin-Gemisch kann gegebenenfalls vor der Metathese-Reaktion einer entsprechenden Behandlung an Adsorber-Schutzbetten, bevorzugt an hochoberflächigen Aluminiumoxiden oder Molsieben, zur Befreiung von störenden Verunreinigungen behandelt werden.

Die aus Schritt b) gegebenenfalls erhaltene Leichtsiederfraktion A, die C₂-C₃-Olefine enthält, wird destillativ in eine Ethen enthaltende Fraktion und eine Propen enthaltende Fraktion getrennt. Die Ethen enthaltende Fraktion wird sodann in den Verfahrensschritt a), d.h. die Metathese, zurückgeführt, und die Propen enthaltende Fraktion wird als Produkt ausgeschleust.

In Schritt d) kann die Trennung in Leichtsiederfraktion B, Mittelsiederfraktion C und Schwersiederfraktion D beispielsweise in einer Trennwandkolonne durchgeführt werden. Hierbei wird die Leichtsiederfraktion B über Kopf, die Mittelsiederfraktion C über einen Mittelaustrag und die Schwersiederfraktion D als Sumpf erhalten.

Um die bei dem flexibel gesteuerten Verfahren anfallenden unterschiedlich großen Mengen an Produkten besser handhaben zu können, ist es jedoch vorteilhaft, eine zweistufige Auftrennung der aus b) erhaltenen Schwersiederfraktion durchzuführen. Vorzugsweise wird die aus b) erhaltene Schwersiederfraktion zunächst destillativ in eine Butene und Butane enthaltende Leichtsiederfraktion B und eine 2-Penten und 3-Hexen enthaltende Hochsiederfraktion getrennt. Die Hochsiederfraktion wird sodann destillativ in die Fraktionen C und D getrennt.

Die Metathesereaktion wird dabei vorzugsweise in Gegenwart von heterogenen, nicht oder nur geringfügig isomerisierungsaktiven Metathesekatalysatoren durchgeführt, die aus der Klasse der auf anorganischen Trägern aufgebrachten Übergangsmetallverbindungen von Metallen der VI.b, VII.b oder VIII.-Gruppe des Periodensystems der Elemente ausgewählt sind.

Bevorzugt wird als Metathesekatalysator Rheniumoxid auf einem Träger, vorzugsweise auf γ-Aluminiumoxid oder auf Al₂O₃/B₂O₃/SiO₂-Mischträgern eingesetzt.

Insbesondere wird als Katalysator Re₂O₇/γ-Al₂O₃ mit einem Rheniumoxid-Gehalt von 1 bis 20 Gew.-%, vorzugsweise 3 bis 15 Gew.-%, besonders bevorzugt 6 bis 12 Gew.-% eingesetzt.

Die Metathese wird bei Flüssigfahrweise vorzugsweise bei einer Temperatur von 0 bis 150°C, besonders bevorzugt 20 bis 80°C sowie einem Druck von 2 x 10⁵ bis 200 x 10⁵ Pa (2 bis 200 bar), besonders bevorzugt 5 x 10⁵ bis 30 x 10⁵ Pa (5 bis 30 bar), durchgeführt.

Wenn die Metathese in der Gasphase durchgeführt wird, beträgt die Temperatur vorzugsweise 20 bis 300°C, besonders bevorzugt 50 bis 200°C. Der Druck beträgt in diesem Fall vorzugsweise 1 x 10⁵ bis 20 x 10⁵ Pa (1 bis 20 bar), besonders bevorzugt 1 x 10⁵ bis 5 x 10⁵ Pa (1 bis 5 bar).

Weiterer Gegenstand der vorliegenden Erfindung ist die Herstellung von C₅/C₆-Olefinen unter Verwendung der Teilschritte (1) bis (4):
(1) Abtrennung von Butadien und acetylenischen Verbindungen durch gegebenenfalls Extraktion von Butadien mit einem Butadien-selektiven Lösungsmittel und nachfolgend erfindungsgemäße Selektivhydrierung von den übrigen enthaltenen Butadienen und acetylenischen Verunreinigungen um einen Reaktionsaustrag zu erhalten, der n-Butene und Isobuten und im wesentlichen keine Butadiene und acetylenischen Verbindungen enthält,
(2) Abtrennung von Isobuten durch Umsetzung des in der vorstehenden Stufe erhaltenen Reaktionsaustrags mit einem Alkohol in Gegenwart eines sauren Katalysators zu einem Ether, Abtrennung des Ethers und des Alkohols, die gleichzeitig oder nach der Veretherung erfolgen kann, um einen Reaktionsaustrag zu erhalten, der n-Butene und gegebenenfalls Oxygenat-Verunreinigungen enthält, wobei gebildeter Ether ausgetragen oder zur Reingewinnung von Isobuten rückgespalten werden kann und dem Veretherungsschritt ein Destillationsschritt zur Abtrennung von Isobuten nachfolgen kann, wobei gegebenenfalls auch eingeschleuste C₃-, i-C₄- sowie C₅-Kohlenwasserstoffe destillativ im Rahmen der Aufarbeitung des Ethers abgetrennt werden können, oder Oligomerisierung oder Polymerisation von Isobuten aus dem in der vorstehenden Stufe erhaltenen Reaktionsaustrag in Gegenwart eines sauren Katalysators, dessen Säurestärke zur selektiven Abtrennung von Isobuten als Oligo- oder Polyisobuten geeignet ist, um einen Strom zu erhalten, der 0 bis 15 % Rest-Isobuten aufweist,
(3) Abtrennen der Oxygenat-Verunreinigungen des Austrags der vorstehenden Schritte an entsprechend ausgewählten Adsorbermaterialien,
(4) Metathesereaktion des so erhaltenen Raffinats II-Stromes wie beschrieben.

Die Selektivhydrierung erfolgt dabei wie zuvor beschrieben.

Die genaue Durchführung der Metathesestufe erfolgt vorzugsweise wie folgt:
Der nach der Veretherung/Polymerisation (bzw. Destillation) erhaltene Raffinat II-Strom wird vorzugsweise an mindestens einem Schutzbett (guard bed), bestehend aus hochoberflächigen Aluminiumoxiden, Kieselgelen, Alumosilikaten oder Molsieben, gereinigt. Das Schutzbett dient hierbei zum Trocknen des C₄-Stroms sowie zur Entfernung von Substanzen, welche als Katalysatorgift im nachfolgenden Metatheseschritt wirken können. Die bevorzugten Adsorbermaterialien sind Selexsorb CD und CDO sowie 3Å- und NaX-Molsiebe (13X). Die Reinigung erfolgt in Trockentürmen bei Temperaturen und Drucken, die so gewählt sind, dass sämtliche Komponenten in der flüssigen Phase vorliegen. Gegebenenfalls wird der Reinigungsschritt zur Feed-Vorwärmung für den nachfolgenden Metatheseschritt eingesetzt.

Der verbleibende Raffinat II-Strom ist annähernd frei von Wasser, Oxygenaten, organischen Chloriden und Schwefelverbindungen. Die Vorgehensweise ist allgemein auf C₄-Ausgangsströme anwendbar.

Bei Durchführung des Veretherungsschritts mit Methanol zur Herstellung von MTBE kann es aufgrund der Bildung von Dimethylether als Nebenkomponente erforderlich sein, mehrere Reinigungsschritte zu kombinieren bzw. hintereinander zu schalten.

Als Metathesekatalysatoren werden literaturbekannte heterogene Rhenium-Katalysatoren, wie Re₂O₇ auf γ-Al₂O₃ oder auf Mischträgern, wie z.B. SiO₂/Al₂O₃, B₂O₃/SiO₂/Al₂O₃ oder Fe₂O₃/Al₂O₃ mit unterschiedlichem Metallgehalt bevorzugt. Der Rheniumoxid-Gehalt beträgt unabhängig vom gewählten Träger zwischen 1 und 20 %, vorzugsweise zwischen 3 und 10 %.

Die Katalysatoren werden im Allgemeinen frisch calziniert eingesetzt und bedürfen keiner weiteren Aktivierung (z.B. durch Alkylierungsmittel). Desaktivierter Katalysator kann durch Abbrennen von Coke-Rückständen bei Temperaturen oberhalb von 400°C im Luftstrom und Abkühlung unter Inertgas-Atmosphäre mehrfach regeneriert werden.

Ein Vergleich der Heterogenkontakte untereinander zeigt, dass Re₂O₇/Al₂O₃ bereits unter sehr milden Reaktionsbedingungen (T = 20 bis 80°C) aktiv ist, während MO₃/SiO₂ (M = Mo, W) erst bei Temperaturen oberhalb von 100 bis 150°C Aktivität entwickelt und demzufolge als Nebenreaktionen C=C-Doppelbindungsisomerisierung auftreten kann.

Ferner sind zu nennen:
- WO₃/SiO₂, präpariert aus (C₅H₅)W(CO)₃Cl und SiO₂ in J. Mol Catal. 1995, 95, 75-83;
- 3-Komponenten-System, bestehend aus [Mo(NO)₂(OR)₂]n, SnEt₄ und AlCl₃ in J. Mol. Catal. 1991, 64, 171-178 und J. Mol. Catal 1989, 57, 207-220;
- Nitridomolybdän (VI)-Komplexe aus hochaktive Präkatalysatoren in J. Organomet. Chem. 1982, 229, C19-C23;
- heterogene SiO₂-geträgerte MoO₃ und WO₃-Katalysatoren in J. Chem. Soc., Faraday Trans. / 1982, 78, 2583-2592;
- geträgerte Mo-Katalysatoren in J. Chem. Soc., Faraday Trans. / 1981, 77, 1763-1777;
- aktive Wolfram-Katalysatorvorstufe in J. Am. Chem. Soc. 1980, 102(21), 6572-6574;
- Acetonitril(pentacarbonyl)wolfram in J. Catal. 1975, 38, 482-484;
- Trichloro(nitrosyl)molybdän(II) als Katalysator-Vorstufe in Z. Chem. 1974, 14, 284-285;
- W(CO)₅PPH₃EtAlCl₂ in J. Catal. 1974, 34, 196-202;
- WCl₆/n-BuLi in J. Catal 1973, 28, 300-303;
- WCl₆/n-BuLi in J. Catal. 1972, 26, 455-458;

FR 2 726 563: O₃ReO[Al(OR)(L)xO]nReO₃ mit R = C₁-C₄₀-Kohlenwasserstoff, n = 1-10, x = 0 oder 1 und L = Solvens,
EP-A-191 0 675, EP-A-129 0 474, BE 899897: Katalysatorsysteme aus Wolfram, 2 substituierten Phenolatresten und 4 anderen Liganden, u.a. einer Halogen-, Alkyl- bzw. Carbengruppe.

FR 2 499 083: Katalysatorsystem aus einem Wolfram-, Molybdän- oder Rhenium-Oxo-Übergangsmetallkomplex mit einer Lewissäure.

US 4,060,468: Katalysatorsystem aus einem Wolframsalz, einer sauerstoffhaltigen aromatischen Verbindung, z.B. 2,6-Dichlorphenol und wahlweise molekularem Sauerstoff.

BE 776,564: Katalysatorsystem aus einem Übergangsmetallsalz, einer metallorganischen Verbindung und einem Amin.

Für die Verbesserung der Cyclusdauer der eingesetzten Katalysatoren, vor allem der geträgerten Katalysatoren, empfiehlt sich der Einsatz einer Feed-Reinigung an Adsorberbetten (guard beds). Das Schutzbett dient hierbei zum Trocknen des C₄-Stroms sowie zur Entfernung von Substanzen, welches als Katalysatorgift im nachfolgenden Metatheseschritt wirken können. Die bevorzugten Adsorbermaterialien sind Selexsorb CD und CDO sowie 3Å- und NaX-Molsiebe (13X). Die Reinigung erfolgt in Trockentürmen bei Temperaturen und Drucken, die bevorzugt so gewählt sind, dass sämtliche Komponenten in der flüssigen Phase vorliegen. Gegebenenfalls wird der Reinigungsschritt zur Feed-Vorwärmung für den nachfolgenden Metatheseschritt eingesetzt. Es kann von Vorteil sein, mehrere Reinigungsschritte miteinander zu kombinieren bzw. hintereinander zu schalten.

Druck und Temperatur im Metatheseschritt sind so gewählt, dass sämtliche Reaktionspartner in der flüssigen Phase vorliegen (üblicherweise = 0 bis 150°C, bevorzugt 20 bis 80°C; p = 2 x 10⁵ bis 200 x 10⁵ Pa (2 bis 200 bar)). Alternativ kann es aber von Vorteil sein, insbesondere bei Feedströmen mit höherem Isobutengehalt, die Umsetzung in der Gasphase durchzuführen und/oder einen Katalysator einzusetzen, der über eine geringere Acidität verfügt.

In der Regel ist die Umsetzung nach 1 s bis 1 h, vorzugsweise nach 30 s bis 30 min beendet. Sie kann kontinuierlich oder diskontinuierlich in Reaktoren, wie Druckgasgefäßen, Strömungsrohren oder Reaktivdestillationsvorrichtungen durchgeführt werden, wobei Strömungsrohre bevorzugt werden.

Weiterer Gegenstand der vorliegenden Erfindung ist die Verwendung von C₄-Olefin-Gemischen zur Herstellung von 2-Penten und/oder 3-Hexen.

Die vorliegende Erfindung wird anhand der nachstehenden Beispiele näher erläutert.

### Beispiele

### Durchführung:

Ein C4-Strom aus einem Steamcracker wird einer dreistufigen Hydrierung unterzogen, wobei in der ersten Hydrierstufe eine Hydrierung an dem erfindungsgemäß vorgesehenen Katalysator durchgeführt wird.

Die Zusammensetzung des Ausgangsstromes sowie die Zusammensetzungen, die aus den jeweiligen Stufen erhalten werden, sind untenstehender Tabelle 1 zu entnehmen. Das Beispiel verdeutlicht, dass durch die Hydrierung an dem erfindungsgemäßen Katalysator sowie durch das erfindungsgemäße Verfahren es möglich ist, den Anteil an 1,2-Dienen in dem Steamcrackeraustrag zu minimieren sowie den 1-Butengehalt, hier auf 50,70 Gew.-%, zu maximieren.

Die jeweiligen Verfahrensbedingungen sind ebenfalls der Tabelle 1 zu entnehmen.

**Tabelle 1:**

| | | Edukt | 1. Stufe | 2: Stufe | Raff2 | 3. Stufe |
|---|---|---|---|---|---|---|
| 1,3-Butadien | [Gew.-%] | 37,8 | 3,5 | 0,15 | 0,1992 | 0,0100 |
| 1-Buten | [Gew.-%] | 17,9 | 39,4 | 38,82 | 51,56 | 50,53 |
| 2-Buten-trans | [Gew.-%] | 6,9 | 15,7 | 18,0 | 23,96 | 24,44 |
| 2-Buten-cis | [Gew.-%] | 4,0 | 8,4 | 9,5 | 12,60 | 12,84 |
| i-Buten | [Gew.-%] | 26,7 | 26,7 | 26,7 | 2 | 2 |
| i-Butan | [Gew.-%] | 1,1 | 1,1 | 1,1 | 1,46 | 1,46 |
| n-Butan | [Gew.-%] | 5,0 | 5 | 5,5 | 7,30 | 7,80 |
| Butenin | [Gew.-%] | 0,3 | <0,0002 | <0,0002 | <0,0002 | <0,0002 |
| Butin | [Gew.-%] | 0,1 | <0,0002 | <0,0002 | <0,0002 | <0,0002 |
| 1,2-Butadien | [Gew.-ppm] | 1900 | 70 | <2 | <2 | <2 |
| Propadien | [Gew.-ppm] | 50 | <2 | <2 | <2 | <2 |
| | | | | | | |
| **Summe 1,2-Diene** | **[Gew.-ppm]** | 1950 | 70 | <4 | | <4 |
| **1,3-Butadien** | **[Gew.-ppm]** | | | | | 100 |
| Gesamt-Buten-Sel. | [%] | | 100,0 | | | |
| 1-Buten-Sel. | [%] | | 62,0 | | | |
| Umsatz | [%] | | 90,74 | 95,71 | | 94,98 |
| 1-B/2-B | | 1,64 | 1,64 | 1,41 | 1,41 | 1,36 |
| 1-Buten-Erhalt | [%] | | | 98,5 | | 98 |
| n-Butan-Bildung | [%] | | 0 | 0,5 | | 0,5 |
| Summe Butene | [%] | | | | 88,11 | 87,80 |
| | | | | | | |
| Katalysator | | | Erf.gemäß | STdT | | StdT |
| Druck | (Barg) 10⁵ Pa | | (20)21 | (15) 16 | | (15) 16 |
| Eintrittstemperatur | ° C | | 50 | 40 | | 45 |
| Frischfeed-Belastung (whsv) | kg/lh | | 3 | 4,4 | | 6 |
| Kreislauf-Menge / Frischfeed-Menge | | | 10 | 4 | | 0 |
| Reaktionsführung | | | adiabatisch | adiabatisch | | adiabatisch |
| Gehalt an Inerten im Wasserstoff | Vol-% | | 10 | 10 | | 0 |

### Durchführung einer Metathese:

Es werden Olefinströme eingesetzt, die nach den in der Beschreibung genannten Verfahren hergestellt wurden, beispielsweise ein Roh-C4-Schnitt durch eine erfindungsgemäße Selektivhydrierung behandelt und danach enthaltenes Isobuten durch Veretherung mittels literaturbekannten Verfahren bis auf einen Restgehalt von < 3 % entfernt.

Der C4-Olefinstrom mit der jeweils angegebenen Zusammensetzung wird zunächst über ein 13X-Molsieb geleitet, um Oxigenate zu entfernen, auf den Reaktionsdruck von 40 x 10⁵ Pa (40 bar) komprimiert, im angegebenen Verhältnis mit frisch zugegebenem Ethen (Messung per Differenzwägung) vermischt sowie der entsprechende C4-Recyclestrom eingestellt. Der C4-Recyclestrom wird dabei so gewählt, dass ein Gesamtbuten-Umsatz von 60 % erreicht wird. Darüber hinaus anfallende C4-Mengen werden aus dem System entfernt, um keine Aufpegelung der Butane zu gestatten (sog. C4-Purge). Der in der 3. Kolonne abgetrennte C5-Recyclestrom wird komplett vor den Reaktor zurückgeführt, um die Kreuzmetathese zwischen 1-Buten und 2-Buten zu unterdrücken. Das Reaktionsgemisch wird in einem 500 ml Rohrreaktor mittels eines nach Literaturangaben hergestellten 10 %igen Re₂O₇-Katalysators (auf Al₂O₃) metathetisiert. Die Reaktionstemperatur ist jeweils angegeben.

Der Austrag wird mittels drei Kolonnen in einen C2/3-, C4-, C5 sowie C6-Strom getrennt, die einzelnen Ströme werden gaschromatographisch analysiert.

Die Bilanzen wurden jeweils für 24 h bei konstanter Reaktionstemperatur erstellt.

### Beispiel 1 (Vergleichsbeispiel, nicht erfindungsgemäß)

Einsatz eines Raffinat II mit 93 ppm 1,3-Butadien und 25 ppm Propadien

### Anlageneinstellungen:

| | |
|---|---|
| Katalysatorbelastung (Gesamtstrom über Reaktor): | 5,0 kg/kg Kat/h |
| Raffinat II: | 870 g/h |
| C4-Recycle: | 630 g/h |
| Ethenfeed: | 65 g/h |

### Zusammensetzung des Feeds

| | |
|---|---|
| 1-Buten: | 52,8 % |
| 2-Buten: | 34,1 % |
| n-Butan: | 9,4 % |
| i-Butan: | 1,9 % |
| i-Buten: | 1,8% |

(Rest besteht aus inerten C3, C4 und C5-Kohlenwasserstoffen).

### Darstellung des Ergebnisses:

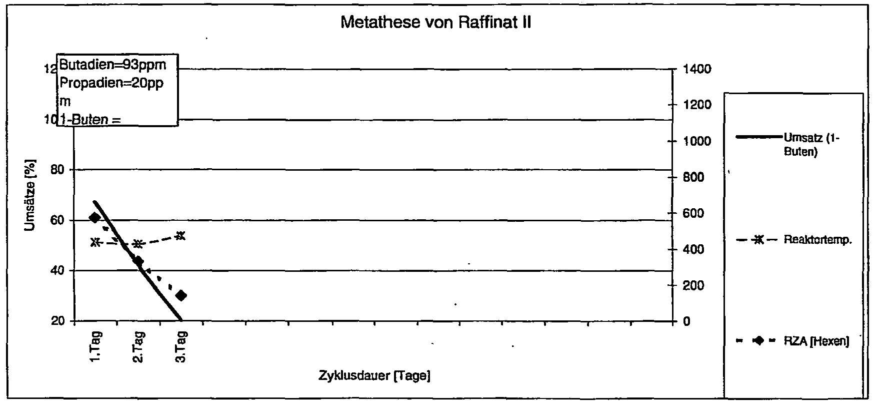

Es findet rasche Desaktivierung statt, die Produktion der Wertprodukte 3-Hexen und Propen sinkt bereits nach drei Tagen auf nahezu Null ab.

### Beispiel 2 (Vergleichsbeispiel, nicht erfindungsgemäß)

Einsatz eines Raffinat II mit 93 ppm 1,3-Butadien und 25 ppm Propadien

### Anlageneinstellungen:

| | |
|---|---|
| Katalysatorbelastung (Gesamtstrom über Reaktor): | 3,0 kg/kg Kat/h |
| Raffinat II: | 500 g/h |
| C4-Recycle: | 400 g/h |
| Ethenfeed: | 40 g/h |

### Zusammensetzung des Feeds:

| | |
|---|---|
| 1-Buten: | 53,3% |
| 2-Buten: | 32,8 % |
| n-Butan: | 9,1 % |
| i-Butan: | 1,9% |
| i-Buten: | 1,8% |

(Rest besteht aus inerten C3, C4 und C5-Kohlenwasserstoffen).

### Darstellung des Ergebnisses:

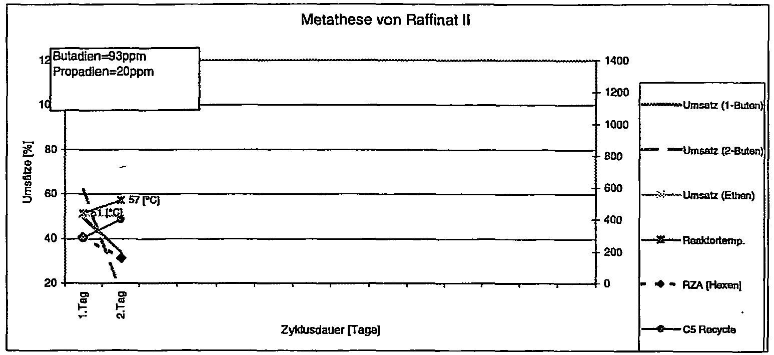

Es findet rasche Desaktivierung statt, die Produktion der Wertprodukte 3-Hexen und Proben sinkt bereits nach zwei Tagen auf nahezu Null ab.

### Beispiel3 (erfindungsgemäß)

Einsatz eines Raffinat II mit 150 ppm 1,3-Butadien, 2 ppm Propadien

### Anlageneinstellungen

| | |
|---|---|
| Katalysatorbelastung: | 5 kg/kg Kat/h |
| Raffinat II: | 930 g/h |
| C4-Recycle: | 560 g/h |
| Ethenfeed: | 70 g/h |

### Zusammensetzung des Feeds

| | |
|---|---|
| 1-Buten: | 51,3% |
| 2-Buten: | 33,3 % |
| n-Butan: | 10,6 % |
| i-Butan: | 2,1 % |
| i-Buten: | 2,2 % |

(Rest besteht aus inerten C3, C4 und C5-Kohlenwasserstoffen).

### Darstellung des Ergebnisses:

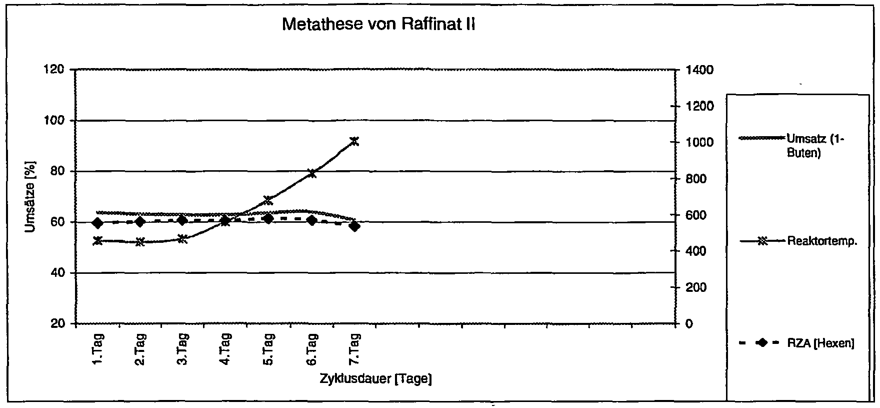

Die Aktivität des Katalysators kann durch geeignete Temperaturerhöhung der Reaktionstemperatur konstant gehalten werden. Die Ausbeute an Wertprodukten ist damit deutlich größer als in den Fällen 1+2.

### Beispiel 4 (erfindungsgemäß)

Einsatz eines Raffinat II mit 62 ppm 1,3-Butadien, 2 ppm Propadien und 8 ppm 1,2-Butadien

### Anlageneinstellungen

| | |
|---|---|
| Katalysatorbelastung: | 6 kg/(kg Kat/h) |
| Raffinat II: | 1120 g/h |
| C4-Recycle: | 650 g/h |
| Ethenfeed: | 98 g/h |

### Zusammensetzung des Feeds

| | |
|---|---|
| 1-Buten: | 51,5% |
| 2-Buten: | 32,4 % |
| n-Butan: | 11,6 % |
| i-Butan: | 2,2 % |
| i-Buten: | 1,8% |

(Rest besteht aus inerten C3, C4 und C5-Kohlenwasserstoffen)

### Darstellung des Ergebnisses:

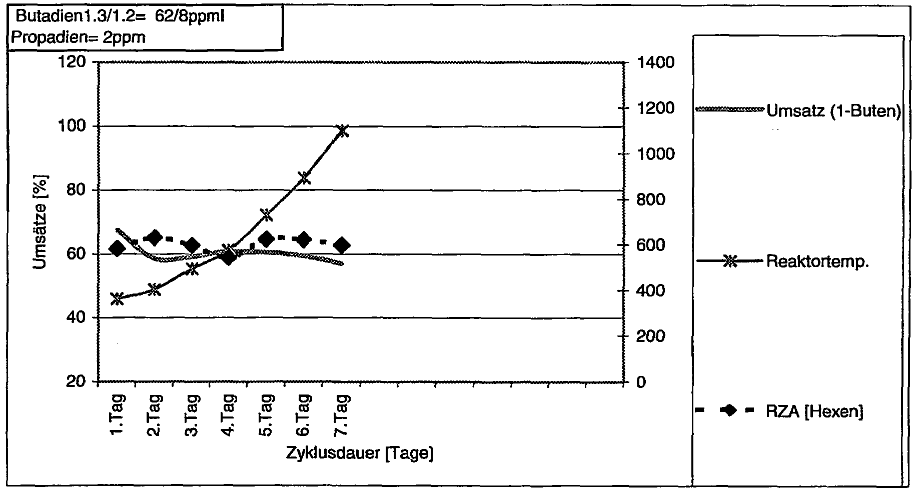

Die Aktivität des Katalysators kann durch geeignete Temperaturerhöhung der Reaktionstemperatur konstant gehalten werden. Die Ausbeute an Wertprodukten ist damit deutlich größer als in den Fällen 1 + 2.

## Patentansprüche

1. C4-Olefin-Gemisch mit einem Gehalt an 1,3-Butadien von 100 bis 500 ppm und einem Gehalt an kumulierten Dienen von 1 bis 10 ppm, **dadurch gekennzeichnet, dass** dass der Gehalt an 1-Buten in dem Gemisch mindestens 30 Gew.-%, bezogen auf das Gemisch, beträgt.

2. C4-Olefin-Gemisch nach Anspruch 1, **dadurch gekennzeichnet, dass** der Gehalt an kumulierten 1,2-Dienen weniger als 10 ppm beträgt.

3. C4-Olefin-Gemisch nach Anspruch 1 oder 2, **dadurch gekennzeichnet, dass** das Verhältnis von 1-Buten zu 2-Butenen im Gemisch 1,2 bis 2,0 beträgt.

4. C4-Olefin-Gemisch nach einem der Ansprüche 1 bis 3, **dadurch gekennzeichnet, dass** das Gemisch 1-Buten, trans-2-Buten, cis-2-Buten, Isobuten, Isobutan und n-Butan enthält

5. Verfahren zur Herstellung eines C4-Olefin-Gemisches mit einem Gehalt an 1,3-Butadien von 100 bis 500 ppm und einem Gehalt an kumulierten Dienen von weniger als 10 ppm durch Hydrierung eines aus Steamcrackern stammenden C4-Stroms, **gekennzeichnet durch** einen Gehalt an kumulierten Dienen von weniger als 10 ppm, **dadurch** gekennzeichnet, dass in der ersten Hydrierstufe die Hydrierung an einem Katalysator durchgeführt wird, der mindestens ein Metall der VIII. Gruppe des Periodensystems der Elemente als Hydriermetall und zusätzlich einen Promotor auf einem oxidischen Träger umfasst, wobei mindestens 80 % des Metalls der VIII. Gruppe des Periodensystems der Elemente in einer Schicht zwischen der Oberfläche des Katalysators und einer Eindringtiefe, die maximal 80 % des Radius des Katalysators, gerechnet von der Oberfläche des Katalysators, entspricht, im Wesentlichen homogen und der Promotor über den gesamten Querschnitt des Katalysators im Wesentlichen homogen verteilt vorliegt.

6. Verfahren nach Anspruch 5, **dadurch gekennzeichnet, dass** die Hydrierung in mindestens zwei Hydrierstufen durchgeführt wird.

7. Verfahren nach einem der Ansprüche 5 und 6, **dadurch gekennzeichnet, dass** der Katalysator einen Durchmesser von 2,5 bis 10 mm aufweist, wobei mindestens 80 % des Metalls der VIII. Gruppe des Periodensystems der Elemente in einer Schicht zwischen der Oberfläche des Schalenkatalysators und einer Eindringtiefe von maximal 1000 µm, gerechnet von der Oberfläche des Katalysators, im Wesentlichen homogen und der Promotor über den gesamten Querschnitt im Wesentlichen homogen verteilt vorliegt.

8. Verfahren nach einem der Ansprüche 5 bis 7, **dadurch gekennzeichnet, dass** der oxidische Träger eine Mischung aus δ-, θ- und α-Aluminiumoxid ist.

9. Verfahren nach einem der Ansprüche 5 bis 8, **dadurch gekennzeichnet, dass** das Metall der VIII. Gruppe des Periodensystems der Elemente Palladium ist.

10. Verfahren nach einem der Ansprüche 5 bis 9, **dadurch gekennzeichnet, dass** der Promotor ein Metall der IB. Gruppe des Periodensystems der Elemente ist.

11. Verfahren nach Anspruch 10, **dadurch gekennzeichnet, dass** das Metall der IB. Gruppe des Periodensystems der Elemente Silber ist.

12. Verfahren nach einem der Ansprüche 5 bis 11, **dadurch gekennzeichnet, dass** das Atomverhältnis zwischen dem Metall der VIII. Gruppe des Periodensystems der Elemente zum Metall der IB. Gruppe des Periodensystems der Elemente 0,1 bis 10 beträgt.

13. Verfahren nach einem der Ansprüche 6 bis 12, **dadurch gekennzeichnet, dass** die Eintrittstemperatur in die zweite Hydrierstufe 20 bis 100°C beträgt.

14. Verfahren nach einem der Ansprüche 6 bis 13, **dadurch gekennzeichnet, dass** das Verhältnis von Wasserstoff zu Butadien 1 bis 3 beträgt.

15. Verfahren nach einem der Ansprüche 6 bis 14, **dadurch gekennzeichnet, dass** der Druck in der zweiten Verfahrensstufe 5 bis 50 barg beträgt.

16. Verfahren zur Herstellung eines 2-Penten und/oder 3-Hexen enthaltenden Olefin-Gemisches durch Umsetzung des C₄-Olefin-Gemischs gemäß einem der Ansprüche 1 bis 4 an einem Metathese-Katalysator.

17. Verfahren nach Anspruch 16, **dadurch gekennzeichnet, dass**
a. in Gegenwart eines Metathese-Katalysators, der mindestens eine Verbindung eines Metalls der VI.b, VII.b oder VIII. Nebengruppe des Periodensystems der Elemente enthält, das C4-Olefin-Gemisch gemäß einem der Ansprüche 1 bis 4 einer Metathese-Reaktion unterzogen wird, im Rahmen derer im C₄-Olefin-Gemisch enthaltene Butene mit Ethen zu einem Ethen, Propen, Butene, 2-Penten, 3-Hexen und Butane enthaltenden Gemisch umgesetzt werden, wobei bezogen auf die Butene bis 0,6 Moläquivalente Ethen eingesetzt werden können;
b. der so erhaltene Ausgangsstrom zunächst destillativ getrennt wird in gegebenenfalls eine C₂-C₃-Olefine enthaltende Leichtsiederfraktion A sowie in eine C₄-C₆-Olefine und Butane enthaltende Schwersiederfraktion;
c. die aus (2) gegebenenfalls erhaltene Leichtsiederfraktion A anschließend destillativ in eine Ethen enthaltende Fraktion und eine Propen enthaltende Fraktion getrennt wird, wobei die Ethen enthaltende Fraktion in den Verfahrensschritt (1) zurückgeführt wird und die Propen enthaltende Fraktion als Produkt ausgeschleust wird;
d. die aus (2) erhaltene Schwersiederfraktion anschließend destillativ in eine Butene und Butane enthaltende Leichtsiederfraktion B, eine 2-Penten enthaltende Mittelsiederfraktion C und in eine 3-Hexen enthaltende Schwersiederfraktion D getrennt wird;
e. wobei die Fraktionen B und gegebenenfalls C vollständig oder teilweise in den Verfahrensschritt (1) zurückgeführt werden und die Fraktionen D und gegebenenfalls C als Produkt ausgeschleust werden.

18. Verfahren nach Anspruch 16, **dadurch gekennzeichnet, dass**
(1) in Gegenwart eines Metathese-Katalysators, der mindestens eine Verbindung eines Metalls der VI.b, VII.b oder VIII. Nebengruppe des Periodensystems der Elemente enthält, das C4-Olefin-Gemisch gemäß einem der Ansprüche 1 bis 3 oder 11 einer Metathese-Reaktion unterzogen wird, im Rahmen derer im C₄-Olefin-Gemisch enthaltene Butene mit Ethen zu einem Ethen, Propen, Butene, 2-Penten, 3-Hexen und Butane enthaltenden Gemisch umgesetzt werden, wobei bezogen auf die Butene bis 0,6 Moläquivalente Ethen eingesetzt werden können;
(2) der so erhaltene Ausgangsstrom zunächst destillativ getrennt wird in eine C₂-Leichtsiederfraktion, die über den Kopf einer Destillationskolonne entfernt wird, eine C₃-Leichtsiederfraktion, die anschließend ebenfalls über Kopf der Destillationskolonne entfernt wird unter Erhalt einer C₄-C₆-Olefine und Butane enthaltende Schwersiederfraktion;
(3) Rückführung der in Verfahrensschritt (2) erhaltenen C2-Leichtiederfraktion in den Verfahrensschritt (1) und Ausschleusung der C3-Leichsiederfraktion als Produkt;
(4) die aus (2) erhaltene Schwersiederfraktion anschließend destillativ in eine Butene und Butane enthaltende Leichtsiederfraktion B, eine 2-Penten enthaltende Mittelsiederfraktion C und in eine 3-Hexen enthaltende Schwersiederfraktion D getrennt wird;
(5) wobei die Fraktionen B und gegebenenfalls C vollständig oder teilweise in den Verfahrensschritt (1) zurückgeführt werden und die Fraktionen D und gegebenenfalls C als Produkt ausgeschleust werden.

19. Verwendung von C4-Olefin-Gemischen gemäß einem der Ansprüche 1 bis 4 zur Herstellung von 2-Penten und/oder 3-Hexen enthaltenden Olefin-Gemischen.

## Claims

1. A C4-olefin mixture having a content of 1,3-butadiene of from 100 to 500 ppm and a content of cumulated dienes of from 1 to 10 ppm, wherein the content of 1-butene in the mixture is at least 30% by weight, based on the mixture.

2. The C4-olefin mixture according to claim 1, wherein the content of cumulated 1,2-dienes is less than 10 ppm.

3. The C4-olefin mixture according to claim 1 or 2, wherein the ratio of 1-butene to 2-butenes in the mixture is from 1.2 to 2.0.

4. The C4-olefin mixture according to any of claims 1 to 3, wherein the mixture comprises 1-butene, trans-2-butene, cis-2-butene, isobutene, isobutane and n-butane.

5. A process for preparing a C4-olefin mixture having a content of 1,3-butadiene of from 100 to 500 ppm and a content of cumulated dienes of less than 10 ppm by hydrogenation of a C4 stream originating from steamcrackers and having a content of cumulated dienes of less than 10 ppm, wherein, in the first hydrogenation stage, the hydrogenation is carried out over a catalyst comprising at least one metal of group VIII of the Periodic Table of the Elements as hydrogenation metal and additionally a promoter on an oxidic support, where at least 80% of the metal of group VIII of the Periodic Table of the Elements is essentially homogeneously distributed in a layer between the surface of the catalyst and a penetration depth corresponding to not more than 80% of the radius of the catalyst, reckoned from the surface of the catalyst, and the promoter is essentially homogeneously distributed over the entire cross section of the catalyst.

6. The process according to claim 5, wherein the hydrogenation is carried out in at least two hydrogenation stages.

7. The process according to either claim 5 or 6, wherein the catalyst has a diameter of from 2.5 to 10 mm, where at least 80% of the metal of group VIII of the Periodic Table of the Elements is essentially homogeneously distributed in a layer between the surface of the coated catalyst and a penetration depth of not more than 1000 µm, reckoned from the surface of the catalyst, and the promoter is essentially homogeneously distributed over the entire cross section.

8. The process according to any of claims 5 to 7, wherein the oxidic support is a mixture of δ-, θ- and α-aluminas.

9. The process according to any of claims 5 to 8, wherein the metal of group VIII of the Periodic Table of the Elements is palladium.

10. The process according to any of claims 5 to 9, wherein the promoter is a metal of group IB of the Periodic Table of the Elements.

11. The process according to claim 10, wherein the metal of group IB of the Periodic Table of the Elements is silver.

12. The process, according to any of claims 5 to 11, wherein the atomic ratio of the metal of group VIII of the Periodic Table of the Elements to the metal of group IB of the Periodic Table of the Elements is from 0.1 to 10.

13. The process according to any of claims 6 to 12, wherein the entry temperature into the second hydrogenation stage is from 20 to 100°C.

14. The process according to any of claims 6 to 13, wherein the ratio of hydrogen to butadiene is from 1 to 3.

15. The process according to any of claims 6 to 14, wherein the pressure in the second process stage is from 5 to 50 bar g.

16. The process for preparing an olefin mixture comprising 2-pentene and/or 3-hexene by reaction of the C₄-olefin mixture according to any of claims 1 to 4 over a metathesis catalyst.

17. The process according to claim 16, wherein
a. the C4-olefin mixture according to any of claims 1 to 4 is subjected in the presence of a metathesis catalyst comprising at least one compound of a metal of transition group VI.b, VII.b or VIII of the Periodic Table of the Elements to a metathesis reaction in which butenes comprised in the C₄-olefin mixture are reacted with ethene to form a mixture comprising ethene, propene, butenes, 2-pentene, 3-hexene and butanes, where up to 0.6 molar equivalents of ethene based on the butenes can be used;
b. the output stream obtained in this way is firstly separated by distillation into optionally a low boiler fraction A comprising C₂-C₃-olefins and a high boiler fraction comprising C₄-C₆-olefins and butanes;
c. the low boiler fraction A optionally obtained from (2) is subsequently separated by distillation into an ethene-comprising fraction and a propene-comprising fraction and the ethene-comprising fraction is recirculated to process step (1) and the propene-comprising fraction is discharged as product;
d. the high boiler fraction obtained from (2) is subsequently separated by distillation into a low boiler fraction B comprising butenes and butanes, an intermediate boiler fraction C comprising 2-pentene and a high boiler fraction D comprising 3-hexene;
e. where the fractions B and optionally C are entirely or partly recirculated to process step (1) and the fractions D and optionally C are discharged as product.

18. The process according to claim 16, wherein
(1)the C4-olefin mixture according to any of claims 1 to 4 is subjected in the presence of a metathesis catalyst comprising at least one compound of a metal of transition group VI.b, VII.b or VIII of the Periodic Table of the Elements to a metathesis reaction in which butenes comprised in the C₄-olefin mixture are reacted with ethene to form a mixture comprising ethene, propene, butenes, 2-pentene, 3-hexene and butanes, where up to 0.6 molar equivalents of ethene based on the butenes can be used;
(2) the output stream obtained in this way is firstly separated by distillation into a C₂-low boiler fraction which is removed via the top of a distillation column, a C₃-low boiler fraction which is subsequently likewise removed via the top of the distillation column to give a high boiler fraction comprising C₄-C₆-olefins and butanes;
(3) recirculation of the C2-low boiler fraction obtained in process step (2) to process step (1) and discharge of the C3-low boiler fraction as product;
(4) the high boiler fraction obtained from (2) is subsequently separated by distillation into a low boiler fraction B comprising butenes and butanes, an intermediate boiler fraction C comprising 2-pentene and a high boiler fraction D comprising 3-hexene;
(5) where the fractions B and optionally C are entirely or partly recirculated to process step (1) and the fractions D and optionally C are discharged as product.

19. The use of C4-olefin mixtures according to any of claims 1 to 4 for preparing olefin mixtures comprising 2-pentene and/or 3-hexene.

## Revendications

1. Mélange d'oléfines en C4 ayant une teneur en 1,3-butadiène de 100 à 500 ppm et une teneur en diènes cumulés de 1 à 10 ppm, **caractérisé en ce que** la teneur en 1-butène du mélange est d'au moins 30 % en poids, par rapport au mélange.

2. Mélange d'oléfines en C4 selon la revendication 1, **caractérisé en ce que** la teneur en 1,2-diènes cumulés est inférieure à 10 ppm.

3. Mélange d'oléfines en C4 selon la revendication 1 ou 2, **caractérisé en ce que** le rapport du 1-butène aux 2-butènes dans le mélange vaut de 1,2 à 2,0.

4. Mélange d'oléfines en C4 selon l'une quelconque des revendications 1 à 3, **caractérisé en ce que** le mélange contient du 1-butène, du trans-2-butène, du cis-2-butène, de l'isobutène, de l'isobutane et du n-butane.

5. Procédé pour la production d'un mélange d'oléfines en C4 ayant une teneur en 1,3-butadiène de 100 à 500 ppm et une teneur en diènes cumulés inférieure à 10 ppm, par hydrogénation d'un courant en C4 provenant de vapocraqueurs, **caractérisé par** une teneur en diènes cumulés inférieure à 10 ppm, **caractérisé en ce que** dans le premier stade d'hydrogénation on effectue l'hydrogénation sur un catalyseur qui comprend sur un support de type oxyde au moins un métal du groupe VIII du système périodique des éléments en tant que métal d'hydrogénation et en outre un promoteur, au moins 80 % du métal du groupe VIII du système périodique des éléments se trouvant répartis de façon pratiquement homogène en une couche entre la surface du catalyseur et une profondeur de pénétration qui correspond à 80 % au maximum du rayon du catalyseur, calculée à partir de la surface du catalyseur, et le promoteur se trouvant réparti de façon pratiquement homogène sur toute la section transversale du catalyseur.

6. Procédé selon la revendication 5, **caractérisé en ce que** l'hydrogénation est effectuée en au moins deux stades d'hydrogénation.

7. Procédé selon l'une quelconque des revendications 5 et 6, **caractérisé en ce que** le catalyseur présente un diamètre de 2,5 à 10 mm, au moins 80 % du métal du groupe VIII du système périodique des éléments se trouvant répartis de façon pratiquement homogène en une couche entre la surface du catalyseur-coquille et une profondeur de pénétration d'au maximum 1 000 µm, calculée à partir de la surface du catalyseur, et le promoteur se trouvant réparti de façon pratiquement homogène sur toute la section transversale.

8. Procédé selon l'une quelconque des revendications 5 à 7, **caractérisé en ce que** le support de type oxyde est un mélange d'oxyde d'aluminium δ, θ et α.

9. Procédé selon l'une quelconque des revendications 5 à 8, **caractérisé en ce que** le métal du groupe VIII du système périodique des éléments est le palladium.

10. Procédé selon l'une quelconque des revendications 5 à 9, **caractérisé en ce que** le promoteur est un métal du groupe IB du système périodique des éléments.

11. Procédé selon la revendication 10, **caractérisé en ce que** le métal du groupe IB du système périodique des éléments est l'argent.

12. Procédé selon l'une quelconque des revendications 5 à 11, **caractérisé en ce que** le rapport atomique entre le métal du groupe VIII du système périodique des éléments au métal du groupe IB du système périodique des éléments vaut de 0.1 à 10.

13. Procédé selon l'une quelconque des revendications 6 à 12, **caractérisé en ce que** la température d'entrée dans le deuxième stade d'hydrogénation vaut de 20 à 100°C.

14. Procédé selon l'une quelconque des revendications 6 à 13, **caractérisé en ce que** le rapport de l'hydrogène au butadiène vaut de 1 à 3.

15. Procédé selon l'une quelconque des revendications 6 à 14, **caractérisé en ce que** la pression dans le deuxième stade du procédé vaut de 5 à 50 bars (manométrique).

16. Procédé pour la production d'un mélange d'oléfines contenant du 2-pentène et/ou du 3-hexène, par mise en réaction du mélange d'oléfines en C₄ selon l'une quelconque des revendications 1 à 4 sur un catalyseur de métathèse.

17. Procédé selon la revendication 16, **caractérisé en ce que**
a. en présence d'un catalyseur de métathèse, qui contient au moins un composé d'un métal du groupe VIb, VIIb ou VIII du système périodique des éléments, on soumet à une réaction de métathèse le mélange d'oléfines en C4 selon l'une quelconque des revendications 1 à 4, dans le cadre de laquelle les butènes contenus dans le mélange d'oléfines en C₄ sont mis en réaction avec de l'éthène pour donner un mélange contenant de l'éthène, du propène, des butènes, du 2-pentène, du 3-hexène et des butanes, jusqu'à 0,6 équivalent molaire d'éthène par rapport aux butènes pouvant être utilisé ;
b. d'abord on fractionne par distillation le courant de départ ainsi obtenu en éventuellement une fraction A à bas point d'ébullition, contenant des oléfines en C₂-C₃, ainsi qu'en une fraction à haut point d'ébullition, contenant des oléfines en C₄-C₆ et des butanes ;
c. puis on fractionne par distillation la fraction A à bas point d'ébullition, éventuellement obtenue à partir de (2), en une fraction contenant de l'éthène et une fraction contenant du propène, la fraction contenant de l'éthène étant renvoyée dans l'étape (1) du procédé et la fraction contenant du propène étant déchargée en tant que produit ;
d. ensuite on fractionne par distillation la fraction à haut point d'ébullition, obtenue à partir de (2), en une fraction B à bas point d'ébullition, contenant des butènes et des butanes, une fraction C à point d'ébullition moyen, contenant du 2-pentène, et une fraction D à haut point d'ébullition, contenant du 3-hexène ;
e. les fractions B et éventuellement C étant partiellement ou totalement renvoyées dans l'étape (1) du procédé et les fractions D et éventuellement C étant déchargées en tant que produit.

18. Procédé selon la revendication 16, **caractérisé en ce que**
(1) en présence d'un catalyseur de métathèse, qui contient au moins un composé d'un métal du groupe VIb, VIIb ou VIII du système périodique des éléments, on soumet à une réaction de métathèse le mélange d'oléfines en C4 selon l'une quelconque des revendications 1 à 4, dans le cadre de laquelle les butènes contenus dans le mélange d'oléfines en C₄ sont mis en réaction avec de l'éthène pour donner un mélange contenant de l'éthène, du propène, des butènes, du 2-pentène, du 3-hexène et des butanes, jusqu'à 0,6 équivalent molaire d'éthène par rapport aux butènes pouvant être utilisé ;
(2) d'abord on fractionne par distillation le courant de départ ainsi obtenu en une fraction à bas point d'ébullition en C₂, qui est éliminée par la tête d'une colonne de distillation, une fraction à bas point d'ébullition en C₃, qui ensuite est également éliminée par la tête de la colonne de distillation, avec obtention d'une fraction à haut point d'ébullition, contenant des oléfines en C₄-C₆ et des butanes ;
(3) on effectue un recyclage de la fraction à bas point d'ébullition en C2, obtenue dans l'étape (2) du procédé, dans l'étape (1) du procédé et une décharge de la fraction à bas point d'ébullition en C3, en tant que produit ;
(4) ensuite on fractionne par distillation la fraction à haut point d'ébullition, obtenue à partir de (2), en une fraction B à bas point d'ébullition, contenant des butènes et des butanes, une fraction C à point d'ébullition moyen, contenant du 2-pentène, et une fraction D à haut point d'ébullition, contenant du 3-hexène ;
(5) les fractions B et éventuellement C étant partiellement ou totalement renvoyées dans l'étape (1) du procédé et les fractions D et éventuellement C étant déchargées en tant que produit.

19. Utilisation des mélanges d'oléfines en C4 selon l'une quelconque des revendications 1 à 4, pour la production de mélanges d'oléfines contenant du 2-pentène et/ou du 3-hexène.
